# DEMANDE DE BREVET EUROPEEN

(11) **EP 1 647 550 A1**
(43) Date de publication de la demande: **19.04.2006**
(21) Numéro de dépôt: 05291520.4
(22) Date de dépôt: 13.07.2005
(51) Int. Cl.: C07D 251/52, C09B 62/085, C09B 62/095, C09B 62/08

(54) **Monomères ou dimères symmétriques azoïques cationiques leur préparation, compositions les comprenant et procédé de coloration de fibres kératiniques**

(30) Priorité: 23.07.2004 FR 0408186
(71) Demandeur: L'OREAL, 75008 Paris (FR)
(72) Inventeur: Greaves, Andrew, 77144 Montevrain (FR); Baril, Bérangère, 92150 Suresnes (FR)
(74) Mandataire: Wattremez, Catherine

(57) **Abrégé**

La présente invention a pour objet des composés de formules (I) et (II) suivantes, ainsi que leur procédés de synthèse : 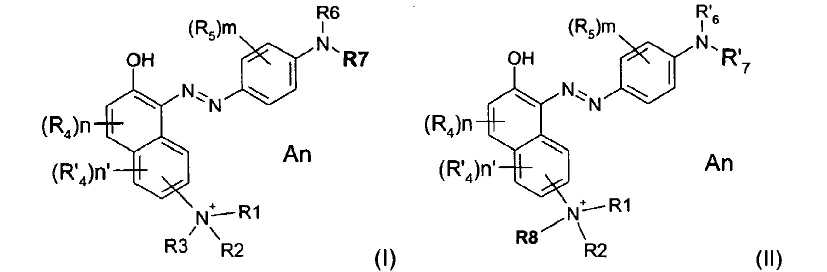 **R7** représentant un groupement choisi parmi les groupes de formules (a) et (b) : 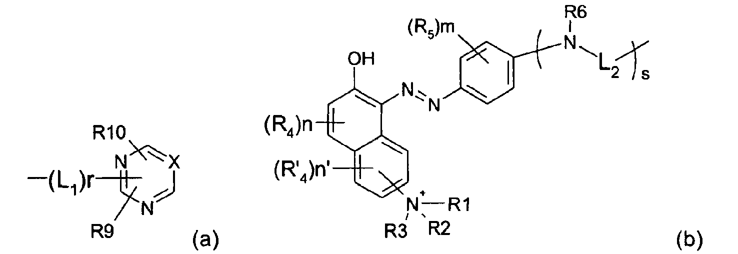 **R8** représente le groupement suivant de formule (c) :

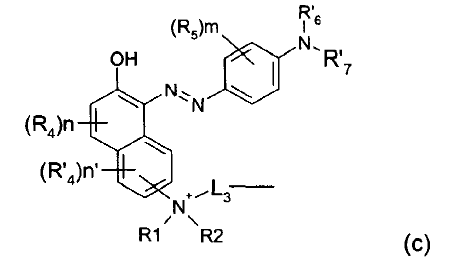

Elle concerne de plus des compositions tinctoriales comprenant lesdits composés, à titre de colorants directs cationiques, ainsi que des procédés de coloration de fibres kératiniques, notamment humaines mettant en oeuvre ces compositions.

## Description

La présente invention a pour objet des composés azoïques cationiques, ou leurs sels d'addition, sous forme de monomère ou de dimère symétrique et leur procédé de préparation. Elle concerne de plus des compositions tinctoriales comprenant lesdits composés, à titre de colorants directs cationiques, ainsi que des procédés de coloration de fibres kératiniques, notamment humaines mettant en oeuvre ces compositions.

Le domaine de la présente invention est plus particulièrement lié à la coloration des matières kératiniques, notamment humaines, et plus particulièrement à celui de la coloration des fibres kératiniques humaines. Par ce terme, on désigne de préférence les cheveux.

De tout temps on a cherché à changer la coloration des cheveux, que ces derniers soient ou non pigmentés, et dans la majeure partie des cas, la coloration souhaitée reste dans des nuances naturelles.

Il existe plusieurs types de coloration parmi lesquels figurent la coloration permanente (ou coloration d'oxydation) et la coloration directe (ou coloration semi-permanente) avec ou sans effet d'éclaircissement des fibres.

Dans le domaine de la coloration d'oxydation, les composés utilisés sont des précurseurs de colorants d'oxydation, plus particulièrement des bases d'oxydation éventuellement associées à un ou plusieurs coupleurs. Ces composés sont des substances incolores ou peu colorées qui, par un processus de condensation oxydative, en présence d'un agent oxydant, conduisent à des composés qui colorent les fibres.

Dans le domaine de la coloration semi-permanente, la composition appliquée sur les fibres comprend en général au moins des colorants directs, qui sont des composés colorants et colorés, et peut éventuellement aussi comprendre au moins un agent oxydant si l'on souhaite obtenir un effet éclaircissant associé à la coloration.

L'une des principales difficultés rencontrées réside dans le fait que pour atteindre de telles colorations, il est nécessaire de mettre en oeuvre des mélanges précis de colorants, tant sur la nature des colorants que sur leurs proportions respectives.

Si le résultat immédiatement après la coloration est satisfaisant, on rencontre toutefois des problèmes d'évolution de la coloration au cours du temps. En effet, les colorants mis en oeuvre dans le mélange ne possèdent pas tous la même affinité pour la fibre, ces différences pouvant être encore accentuées par l'état de sensibilisation des fibres. Ils n'ont pas non plus la même résistance vis-à-vis des facteurs extérieurs comme notamment les rayons ultraviolets, ni la même résistance vis-à-vis des traitements mis en oeuvre sur les fibres, comme par exemple les shampooings. Cela se traduit souvent par un virage de la couleur plus ou moins prononcé, dû au départ de l'un ou plusieurs des colorants présents.

Il existe des colorants directs permettant d'accéder, à eux seuls, à des colorations naturelles. Parmi ces colorants directs on peut citer notamment le colorant Basic Brown16, (Color Index 12250) ou encore le colorant Basic Brown 17 (Color Index12251).

Le problème est que ces colorants ont une ténacité modérée vis-à-vis des shampooings.

La présente invention a donc pour objet de proposer des composés qui puissent être utilisés comme colorants directs pour la teinture de matières kératiniques humaines, notamment des fibres, qui permettent d'obtenir des colorations naturelles, par exemple les bruns, sans qu'il soit nécessaire de mettre en oeuvre des mélanges de colorants divers, et dont la ténacité est améliorée par rapport aux colorants Basic Brown 16 et 17 mentionnés ci-dessus.

Ces buts et d'autres sont atteints par la présente invention qui a donc pour premier objet des composés de formules (I) et (II) suivantes, et leurs sels d'addition : 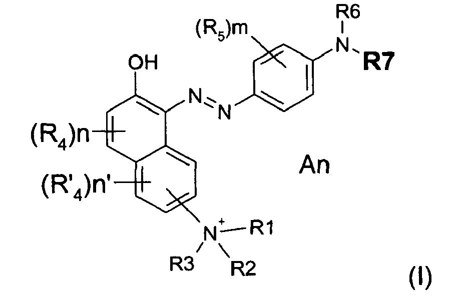 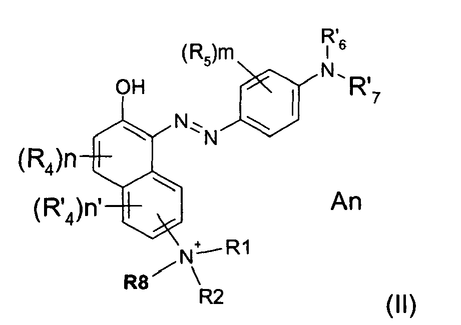 Formules dans lesquelles :
**R1, R2, R3,** identiques ou différents, représentent :
   - un radical alkyle en C1-C4, linéaire ou ramifié, substitué ou non par au moins :
      o un groupement hydroxyle ;
      o un groupement alcoxy, linéaire ou ramifié, en C1-C4 ;
      o un groupement amino ;
      o un groupement amino substitué par un ou deux radicaux, identiques ou différents, alkyle en C1-C4 linéaires ou ramifiés, hydroxyalkyle en C1-C4 linéaires ou ramifiés, deux des radicaux R1, R2 ou R3 pouvant éventuellement former un hétérocycle à 5 ou 6 chaînons, saturé ou insaturé, comprenant éventuellement un autre hétéroatome choisi parmi l'oxygène, l'azote, et éventuellement substitué ;
**R4, R'4, R5**, identiques ou différents, représentent :
   - un atome d'halogène, de préférence le chlore, le fluor ;
   - un groupement hydroxyle ;
   - un groupement nitro, cyano ;
   - un radical alkyle, linéaire ou ramifié, en C1-C4, éventuellement porteur d'au moins un groupement hydroxyle, au moins un groupement alcoxy linéaire ou ramifié en C1-C4, au moins un groupement amino et/ou au moins un groupement amino substitué par un ou deux radicaux alkyles, identiques ou non, linéaires ou ramifiés en C1-C4 ;
   - un radical alcoxy, linéaire ou ramifié, en C1-C4 ;
   - un radical amino ;
   - un radical amino substitué par un ou deux radicaux, identiques ou différents,. choisis parmi les radicaux alkyle en C1-C4, linéaires ou ramifiés, éventuellement substitués par un ou plusieurs radicaux choisis parmi :
      ■ un groupement hydroxyle,
      ■ un groupement amino,
      ■ un groupement amino substitué par un ou deux radicaux, identiques ou différents, linéaires ou ramifiés, alkyle en C1-C4, hydroxyalkyle en C1-C4 ;
      ■ un groupement amino substitué dont les radicaux forment un hétérocycle à 5 ou 6 chaînons, saturé ou insaturé, comprenant éventuellement autre hétéroatome choisi parmi l'oxygène, l'azote ; ledit hétérocycle étant éventuellement substitué ;
   - un groupement -SO₂NHR dans lequel R représentant un atome d'hydrogène, un radical alkyle en C1-C4, éventuellement substitués par un ou plusieurs radicaux hydroxyle, amino, amino substitué par un ou deux radicaux, identiques ou différents, alkyle en C1-C4 linéaires ou ramifiés, hydroxyalkyle en C1-C4 linéaires ou ramifiés
deux radicaux **R5** portés par deux atomes de carbone adjacents peuvent également former avec l'atome de carbone auquel chacun est rattaché, un cycle aromatique à 6 chaînons éventuellement substitué ;
**R6, R'6, R'7,** identiques ou différents, représentent :
   ■ un atome d'hydrogène,
   ■ un radical alkyle, linéaire ou ramifié en C1-C8, substitué ou non par un groupement hydroxyle, alcoxy linéaire ou ramifié en C1-C4, amino, amino substitué par un ou deux radicaux, identiques ou différents, alkyle linéaire ou ramifié en C1-C4, hydroxyalkyle linéaire ou ramifié en C1-C4 ; interrompu ou non par un hétéroatome choisi parmi l'oxygène, l'azote ou par un groupe comprenant un hétéroatome tel que de préférence CO, SO₂ ;
   ■ les radicaux **R'6 et R'7** pouvant éventuellement former un hétérocycle à 5 ou 6 chaînons, saturé ou insaturé, comprenant éventuellement autre hétéroatome choisi parmi l'oxygène, l'azote ; ledit hétérocycle étant éventuellement substitué ;
**R7** représente un groupement choisi parmi : 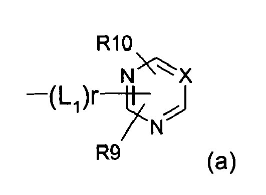 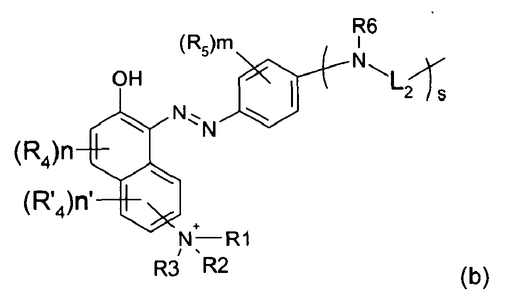
**R8** représente le groupement suivant : 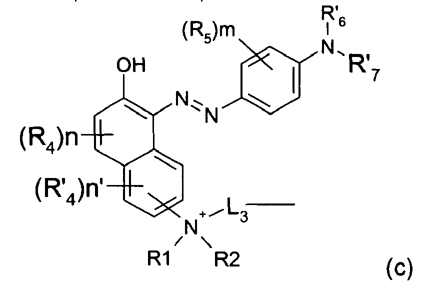
**R1, R2, R3, R4, R'4, R5, R6, R'6, R'7,** dans les formules (a), (b) et (c) ayant la même signification que précédemment ;
**R9, R10,** identiques ou non, représentent :
   - un atome d'hydrogène,
   - un atome d'halogène, de préférence le chlore, le fluor,
   - un groupement nitro, cyano,
   - un radical alkyle, linéaire ou ramifié, en C1-C4, éventuellement porteur d'au moins un groupement hydroxyle, au moins un groupement alcoxy linéaire ou ramifié en C1-C4, au moins un groupement amino et/ou au moins un groupement amino substitué par un ou deux radicaux alkyles, identiques ou non, linéaires ou ramifiés en C1-C4 ;
   - un groupement hydroxyle,
   - un radical alcoxy, linéaire ou ramifié, en C1-C4,
   - un radical amino,
   - un radical amino substitué par un ou deux radicaux, identiques ou différents, choisis parmi les radicaux alkyle en C1-C6, linéaires ou ramifiés, éventuellement substitués par un ou plusieurs radicaux amino, hydroxyle, ammonium ou alkyl(C1-C4)ammonium, imidazolium, pyrazolium, ou pyridinium, amino substitué par un ou deux radicaux, identiques ou différents, alkyle en C1-C4 linéaires ou ramifiés, alcoxy en C1-C4 linéaires ou ramifiés,

   - un radical amino substitué dont les radicaux forment un hétérocycle à 5 ou 6 chaînons, saturé ou insaturé, comprenant éventuellement autre hétéroatome choisi parmi l'oxygène, l'azote ; ledit hétérocycle étant éventuellement substitué ;
   - **R9, R10,** identiques ou non, ne pouvant représenter simultanément un atome de chlore ou de fluor ;
**X** représente un atome d'azote, -CR11 ; **R11** représentant :
   - un atome d'hydrogène
   - un atome d'halogène, de préférence le chlore, le fluor,
   - un groupement nitro, cyano,
   - un radical alkyle, linéaire ou ramifié, en C1-C4, éventuellement porteur d'au moins un groupement hydroxyle, au moins un groupement alcoxy linéaire ou ramifié en C1-C4, au moins un groupement amino et/ou au moins un groupement amino substitué par un ou deux radicaux alkyles, identiques ou non, linéaires ou ramifiés en C1-C4,;
   - un radical alcoxy, linéaire ou ramifié, en C1-C4,
   - un radical amino,
   - un groupement hydroxyle,
   - un radical amino substitué par un ou deux radicaux, identiques ou différents, choisis parmi les radicaux alkyle en C1-C6, linéaires ou ramifiés, éventuellement substitués par un ou plusieurs radicaux amino, hydroxyle, amino substitué par un ou deux radicaux, identiques ou différents, alkyle en C1-C4 linéaires ou ramifiés, hydroxyalkyle en C1-C4 linéaires ou ramifiés,
   - un radical amino substitué dont les radicaux forment un hétérocycle à 5 ou 6 chaînons, saturé ou insaturé, comprenant éventuellement autre hétéroatome choisi parmi l'oxygène, l'azote ; ledit hétérocycle étant éventuellement substitué ;
**L1,** cationique ou non, représente une chaîne hydrocarbonée en C₂-C₂₀, linéaire ramifiée ou cyclique, saturée ou insaturée, éventuellement interrompue ou terminée par un groupement comprenant un ou plusieurs hétéroatomes, éventuellement interrompue par un ou plusieurs (hétéro)cycles, aromatiques ou non, substitués ou non ;
**L2,** cationique ou non, représente :
   - une chaîne hydrocarbonée en C₁-C₂₀, linéaire ramifiée ou cyclique, saturée ou insaturée, éventuellement interrompue ou terminée par un groupement comprenant un ou plusieurs hétéroatomes, éventuellement interrompue ou terminée par un ou plusieurs (hétéro)cycles, aromatiques ou non éventuellement substitués ;
   - un radical (hétéro)cyclique, aromatique ou non, éventuellement rattaché aux atomes d'azote au moyen d'un groupement comprenant au moins un hétéroatome, de préférence un groupement CO, SO₂ ;
**L3,** cationique ou non, représente une chaîne hydrocarbonée en C₂-C₂₀, linéaire ramifiée ou cyclique, saturée ou insaturée, éventuellement interrompue ou terminée par un groupement comprenant un ou plusieurs hétéroatomes, éventuellement interrompue par un ou plusieurs (hétéro)cycles, aromatiques ou non éventuellement substitués ;
le coefficient **n** étant inférieur ou égal à 2 ;
le coefficient **n'** étant inférieur ou égal à 3 ;
le coefficient **m** étant inférieur ou égal à 4 ;
le coefficient **r** étant égal à 0 ou 1 ;
le coefficient **s** étant égal à 0 ou 1 ;
**An** représente un ou plusieurs contre ions cosmétiquement acceptables permettant d'assurer l'électroneutralité des composés de formule (I) et (II) ou de leurs sels d'addition.

La présente invention a de plus pour objet des procédés de synthèse de tels composés.

L'invention concerne de plus des compositions tinctoriales comprenant dans un milieu cosmétiquement acceptable, au moins un composé de formule (I) ou (II), ou leurs sels d'addition, en tant que colorant direct.

Elle a par ailleurs pour objet un procédé de coloration de fibres kératiniques dans lequel on applique sur des fibres sèches ou humides, une composition tinctoriale précitée, avec ou sans rinçage final.

Mais d'autres caractéristiques et avantages de la présente invention apparaîtront plus clairement à la lecture de la description et des exemples qui vont suivre.

Dans ce qui va suivre et à moins qu'une autre information ne soit donnée, les bornes délimitant un domaine de valeurs sont comprises comme faisant partie de ce domaine.

De plus, les dimères de formule (I) dans lesquels **R7** est représenté par la formule (b), sont dits 'symétriques' dans les cas suivants :

Avec s égal à 1, lorsque les radicaux R1, R2, R3, R4, R'4, R5, R6 de la formule (I) identiques ou non, et les radicaux R1, R2, R3, R4, R'4, R5, R6, identiques ou non, de la formule (b), sont les mêmes et sont placés dans les mêmes positions, dans la formule (I) et dans la formule (b).

Avec s égal à 0, lorsque les radicaux R1, R2, R3, R4, R'4, R5, de la formule (I) identiques ou non, et les radicaux R1, R2, R3, R4, R'4, R5, identiques ou non, de la formule (b), sont les mêmes et sont placés dans les mêmes positions, dans la formule (I) et dans la formule (b).

Les dimères de formule (II) avec R8 représentée par la formule (c), sont dits 'symétriques' lorsque les radicaux R1, R2, R4, R'4, R5, R'6, R'7, identiques ou non, de la formule (II) et les radicaux R1, R2, R4, R'4, R5, R'6, R'7, identiques ou non, de la formule (c) sont les mêmes et sont placés dans les mêmes positions, dans la formule (II) et dans la formule (c).

En d'autres termes, les radicaux R1 de la formule (I), R2 de la formule (I), R3 de la formule (I), R4 de la formule (I), etc., sont respectivement les mêmes que les radicaux R1 de la formule (II), R2 de la formule (II), R3 de la formule (II), R4 de la formule (II), etc. De plus ces radicaux sont situés respectivement dans la même position d'un cycle à l'autre ; les valeurs des coefficients n, n' et m respectivement, étant identiques d'une formule à l'autre.

De plus, dans les formules (I), (II), (b) et (c), lorsque les coefficients n, n' et m définissant le nombre de substituants portés par les noyaux aromatiques, ne prennent pas leur valeur maximale, alors l'atome de carbone non substitué porte un atome d'hydrogène.

Enfin, dans la suite, et à moins d'une indication différente, un radical cyclique ou hétérocyclique est dit 'substitué' lorsqu'il porte au moins un radical choisi parmi les groupements :
- hydroxyle,
- alkyle linéaires ou ramifiés en C1-C4,
- hydroxyalkyle linéaires ou ramifiés en C1-C4,
- alcoxy linéaires ou ramifiés en C1-C4,
- alkylamide linéaires ou ramifiés en C1-C4,
- amino,
- amino substitués par un ou deux radicaux alkyle en C1-C4 ou alcoxy en C1-C4, identiques ou différents, linéaires ou ramifiés,
- un groupement ammonium ou alkyl(C1-C4)ammonium, imidazolium, pyrazolium, pyridinium.
- un groupement -SO₂NHR ou -NHRSO₂R', avec R représentant un atome d'hydrogène, un radical alkyle linéaire ou ramifié en C1-C4 éventuellement substitué par un ou plusieurs radicaux hydroxyle, amino, amino substitués par un ou deux radicaux, identiques ou différents, alkyle en C1-C4 linéaires ou ramifiés, hydroxyalkyle en C1-C4 linéaires ou ramifiés et R' représentant un radical alkyle linéaire ou ramifié en C1-C4 éventuellement substitué.

Comme indiqué auparavant, la présente invention concerne des composés de formules (I) et (II) détaillées précédemment.

Plus particulièrement, dans les formules (I) et (II), les radicaux **R1, R2, R3,** identiques ou différents, représentent un radical alkyle en C1-C4, linéaire ou ramifié, substitué ou non par au moins un groupement hydroxyle, un radical alcoxy linéaire ou ramifié en C1-C4, un radical amino, un radical amino substitué par un ou deux radicaux, identiques ou différents, alkyle en C1-C4, linéaires ou ramifiés, hydroxyalkyle en C1-C4, linéaires ou ramifiés.

Conformément à un mode de réalisation préféré, les radicaux **R1, R2, R3,** identiques ou différents, représentent un radical alkyle en C1-C4, linéaire substitué ou non par un groupement hydroxyle.

En ce qui concerne les radicaux **R4, R'4, R5,** des formules (I) et (II), ces derniers, identiques ou différents, représentent plus particulièrement :
- un atome d'halogène, de préférence le chlore,
- un groupement hydroxyle,
- un groupement nitro,
- un radical alkyle, linéaire ou ramifié, en C1-C4, éventuellement substitué par un ou plusieurs groupements hydroxyle, alcoxy en C1-C4 linéaires ou ramifiés,
- un radical alcoxy, linéaire ou ramifié, en C1-C4,
- un radical amino,
- un radical amino substitué par un ou deux radicaux, identiques ou différents, choisis parmi les radicaux alkyle en C1-C4, linéaires ou ramifiés, éventuellement substitués par un ou plusieurs groupements hydroxyle, radicaux amino, radicaux amino substitués par un ou deux radicaux, identiques ou différents, linéaires ou ramifiés, alkyle en C1-C4, hydroxyalkyle en C1-C4, par un groupement ammonium ou alkyl(C1-C4)ammonium, par un groupement -SO₂NHR dans lequel R représente un atome d'hydrogène, un radical alkyle en C1-C4,
- un groupement -SO₂NHR dans lequel R représente un atome d'hydrogène, un radical alkyle en C1-C4.

Conformément à un mode de réalisation préféré, les radicaux **R4, R'4, R5,** représentent :
- un atome d'halogène, de préférence le chlore,
- un groupement hydroxyle,
- un groupement nitro,
- un radical alkyle, linéaire en C1-C2, éventuellement substitué par un ou plusieurs groupements hydroxyle,
- un radical alcoxy, linéaire en C1-C2.

Selon une variante de l'invention, les radicaux **R6, R'6, R'7,** identiques ou différents, représentent un atome d'hydrogène ; un radical alkyle en C1-C4, linéaire ou ramifié, substitué ou non par un groupement hydroxyle, par un groupement alcoxy en C1-C4.

De préférence, les radicaux **R6, R'6, R'7,** identiques ou différents, représentent un atome d'hydrogène, un radical alkyle linéaire en C1-C4.

Dans le cas des radicaux **R9, R10,** identiques ou non, ceux-ci représentent avantageusement :
- un atome d'hydrogène;
- un atome d'halogène, de préférence le chlore, le fluor;
- un groupement nitro, cyano ;
- un groupement hydroxyle,
- un radical alcoxy, linéaire ou ramifié, en C1-C4,
- un radical amino,
- un radical amino substitué par un ou deux radicaux, identiques ou différents, choisis parmi les radicaux alkyle en C1-C6, linéaires ou ramifiés, éventuellement substitués par un ou plusieurs groupements hydroxyle, radicaux amino, radicaux amino substitués par un ou deux radicaux, identiques ou différents, linéaires ou ramifiés, alkyle en C1-C4, hydroxyalkyle en C1-C4, un groupement ammonium ou alkyl(C1-C4)ammonium, imidazolium, pyrazolium, ou pyridinium ,
- un radical amino substitué dont les radicaux forment un hétérocycle à 5 ou 6 chaînons, insaturé, comprenant éventuellement autre hétéroatome choisi parmi l'oxygène, l'azote ; ledit hétérocycle étant éventuellement substitué.

De préférence les radicaux **R9, R10,** identiques ou non, représentent :
- un atome d'hydrogène;
- un atome d'halogène, de préférence le chlore, le fluor,
- un groupement hydroxyle,
- un radical amino,
- un radical amino substitué par un ou deux radicaux, identiques choisis parmi les radicaux alkyle en C1-C4, linéaires, éventuellement substitués par un hydroxyle, amino substitué par un ou deux radicaux, identiques, alkyle en C1-C4 linéaires, un groupement ammonium ou alkyl(C1-C4)ammonium ou imidazolium.
- un radical amino substitué dont les radicaux forment un hétérocycle à 5 ou 6 chaînons, insaturé, comprenant éventuellement un azote ; ledit hétérocycle étant éventuellement substitué.

Le radical **R11** représente plus spécialement :
- un atome d'hydrogène;
- un atome d'halogène, de préférence le chlore, le fluor.
- un groupement nitro, cyano,
- un groupement hydroxyle,
- un radical alcoxy, linéaire ou ramifié, en C1-C4,
- un radical amino,
- un radical amino substitué par un ou deux radicaux, identiques ou différents, choisis parmi les radicaux alkyle en C1-C6, linéaires ou ramifiés, éventuellement substitués par un ou plusieurs groupements hydroxyle, radicaux amino, radicaux amino substitués par un ou deux radicaux, identiques ou différents, linéaires ou ramifiés, alkyle en C1-C4, hydroxyalkyle en C1-C4;
- un radical amino substitué dont les radicaux forment un hétérocycle à 5 ou 6 chaînons, insaturé, comprenant éventuellement autre hétéroatome choisi parmi l'oxygène, l'azote ; ledit hétérocycle étant éventuellement substitué.

Il est à noter que lorsque deux radicaux rattachés à un même atome d'azote, sont liés entre de manière à former un hétérocycle, ce dernier est de préférence choisi parmi les hétérocycles pyrrolidine, pipéridine, homopipéridine, pipérazine, homopipérazine, diazépane. De plus l'hétérocycle peut éventuellement être substitué.

A titre d'exemples plus particuliers, l'hétérocycle est choisi parmi la pyrrolidine, la 3-hydroxypyrrolidine, la 3-aminopyrrolidine, la 3-N,N-diméthylaminopyrrolidine, la 3-triméthylammoniumpyrrolidine, la 3-acétamidopyrrolidine, la 3-(méthylsulfonylamino)-pyrrolidine, la proline, la 3-hydroxyproline, la pipéridine, l'hydroxypipéridine, l'homopipéridine, le diazépane, N-méthyl homopipérazine, la N β-hydroxyéthylhomopipérazine.

Conformément à un mode de réalisation plus particulier de l'invention, dans le cas où le coefficient r est égal à 1, les groupements **L1, L3,** représentent :
- une chaîne alkylène en C1-C20, linéaire ou ramifiée, substituée ou non, éventuellement interrompue par au moins un atome d'oxygène, par au moins un groupement -NR- avec R représentant un atome d'hydrogène, un radical alkyle, linéaire ou ramifié en C1-C4, hydroxyalkyle, linéaire ou ramifié en C1-C4, par au moins un groupement -N⁺(R')(R")- avec R', R", identiques ou non, représentant un radical alkyle, linéaire ou ramifié en C1-C4, hydroxyalkyle, linéaire ou ramifié en C1-C4, par au moins un groupement carbonyle, par au moins un (hétéro)cycle, aromatique ou non, cationique ou non, éventuellement substitué ; éventuellement terminée par un groupement carbonyle.

Au cas où le groupement **L1 ou L3** représente une chaîne alkylène en C1-C20, de préférence en C1-C8, interrompue par au moins un (hétéro)cycle, aromatique ou non, cationique ou non éventuellement substitué ; ledit hétérocycle est choisi parmi les hétérocycles pipérazine, homopipérazine, diazépane, pyrrolidine, (homo)pipéridine, triazine, pipérazinium, homopipérazinium, imidazolium.
- De préférence, **L1, L3,** représentent une chaîne alkylène en C1-C8, linéaire, substituée ou non ; éventuellement interrompue par au moins un atome d'oxygène, par au moins un groupement -NR- avec R représentant un atome d'hydrogène, un radical alkyle, linéaire ou ramifié en C1-C4, hydroxyalkyle, linéaire ou ramifié en C1-C4, par au moins un groupement -N⁺(R')(R")- avec R', R", identiques ou non, un radical alkyle, linéaire ou ramifié en C1-C4, hydroxyalkyle, linéaire ou ramifié en C1-C4, par au moins un hétérocycle éventuellement substitué choisi parmi la pipérazine, la pyrrolidine, la triazine, l'imidazolium ; éventuellement terminée par un groupement carbonyle.

Selon une variante encore plus particulière de l'invention, **L1, L3,** représentent une chaîne alkylène en C1-C8, linéaire, éventuellement interrompue par au moins un atome d'oxygène, au moins un groupement -NR- avec R représentant un atome d'hydrogène, un radical alkyle, linéaire en C1-C4, au moins un groupement -N(R')(R")⁺- avec R', R", identiques, un radical alkyle, linéaire en C1-C4.

Selon un autre mode de réalisation particulier de l'invention, le coefficient r est égal à 0.

Conformément à un mode de réalisation plus particulier de l'invention, le coefficient s est égal à 1 et le groupement **L2**, représente :
- une chaîne alkylène en C1-C20, linéaire ou ramifiée, substituée ou non, éventuellement interrompue par au moins un atome d'oxygène, au moins un groupement -NR- avec R représentant un atome d'hydrogène, un radical alkyle, linéaire ou ramifié en C1-C4, hydroxyalkyle, linéaire ou ramifié en C1-C4, au moins un groupement -N(R')(R")⁺- avec R', R", identiques ou non, un radical alkyle, linéaire ou ramifié en C1-C4, hydroxyalkyle, linéaire ou ramifié en C1-C4, éventuellement interrompue ou terminée par au moins un groupement carbonyle, au moins un (hétéro)cycle, cationique ou non, aromatique ou non, éventuellement substitué ;
- un phénylène ;
- un hétérocycle à 6 ou 7 chaînons, saturé ou non, comprenant au moins deux hétéroatomes, le radical R6 et l'atome d'azote qui le porte étant engagés dans ledit hétérocycle.

Au cas où le groupement **L2** représente une chaîne alkylène en C1-C20, de préférence en C1-C8, interrompue ou terminée par au moins un (hétéro)cycle, aromatique ou non, ce dernier est choisi parmi les hétérocycles pipérazine, homopipérazine, diazépane, triazine ; lesdits hétérocycles étant éventuellement substitués.

De préférence, **L2** représente :
- une chaîne alkylène en C1-C8, linéaire, substituée ou non, éventuellement interrompue par au moins un atome d'oxygène, au moins un groupement -NR-avec R représentant un atome d'hydrogène, un radical alkyle, linéaire ou ramifié en C1-C4, hydroxyalkyle, linéaire ou ramifié en C1-C4, au moins un groupement - N(R')(R")⁺- avec R', R", identiques ou non, un radical alkyle, linéaire ou ramifié en C1-C4, hydroxyalkyle, linéaire ou ramifié en C1-C4, pipérazinium, homopipérazinium, imidazolium éventuellement terminée par au moins un groupement carbonyle, au moins un hétérocycle, aromatique ou non, éventuellement substitué;
- un phénylène ;
- avec le radical R6 et l'atome d'azote qui le porte, un hétérocycle pipérazine ou homopipérazine non substitué.

Selon une variante encore plus préférée de l'invention, le groupe **L2** représente :
- une chaîne alkylène en C1-C8, linéaire, éventuellement interrompue par au moins un atome d'oxygène, au moins un groupement -NR- avec R représentant un atome d'hydrogène, un radical alkyle, linéaire en C1-C4, au moins un groupement -N⁺(R')(R")- avec R', R", identiques, un radical alkyle, linéaire en C1-C4, imidazolium, éventuellement terminée par au moins un hétérocycle, aromatique ou non, éventuellement substitué, de préférence triazine éventuellement substitué ;
- un phénylène ;
- avec le radical R6 et l'atome d'azote qui le porte, un hétérocycle pipérazine ou homopipérazine non substitué.

Selon un mode de réalisation particulier de l'invention, le groupe **L2** représente un groupement de formule (d) suivante : 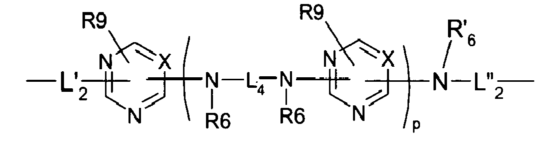 formule dans laquelle :
**R1, R2, R3, R4, R5, R6, R'**_{**6**}**, R9, X,** ayant la même signification que précédemment ;
**L'2, L"2,** identiques ou non, représentent une liaison simple, chaîne alkylène en C1-C6; L'2 étant rattaché au groupement NR6,
**L4** représente une chaîne alkylène en C1-C20, linéaire ou ramifiée, substituée ou non, éventuellement interrompue par au moins un atome d'oxygène, par au moins un groupement -NR- avec R représentant un atome d'hydrogène, un radical alkyle, linéaire ou ramifié en C1-C4, hydroxyalkyle, linéaire ou ramifié en C1-C4, par au moins un groupement -N⁺(R')(R")- avec R', R", identiques ou non, représentant un radical alkyle, linéaire ou ramifié en C1-C4, hydroxyalkyle, linéaire ou ramifié en C1-C4, le coefficient p étant égal à 0 ou 1.

Les composés de formule (I) et (II) et leurs sels étant des composés cationiques, l'électroneutralité de ces composés est satisfaite par la présence d'un ou plusieurs contre ion **An,** identiques ou différents, cosmétiquement acceptables. Le ou les contre-ions sont habituellement choisis parmi les sels d'acides minéraux comme par exemple les chlorures, les bromures, les iodures, les sulfates, hydrogénosulfates, les phosphates ; parmi les sels d'acides organiques comme les formiates, les acétates, les citrates, les succinates, les tartrates, les lactates, les tosylates, les mésylates, les benzènesulfonates, les alkylsulfates comme les méthyl- ou éthyl- sulfates et, ou leurs mélanges.

Quant aux sels d'additions des composés de formules (I) et (II), ces derniers sont choisis parmi les sels d'addition avec un acide correspondant aux sels qui viennent d'être listés. Il peuvent aussi correpsondre à ddes sls d'addition avec des bases telles que, par exemple, la soude, la potasse, l'ammoniaque, les amines ou les alcanolamines.

Conformément à une première variante particulière de l'invention, les composés correspondent à la formule suivante (III) ou ses sels : 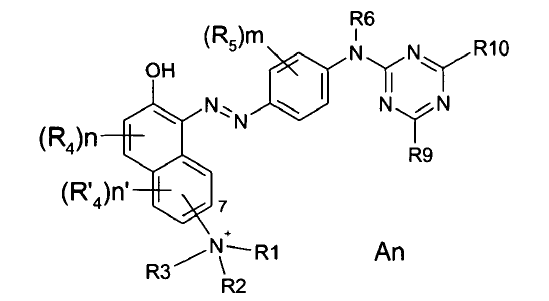 Formule dans laquelle les radicaux **R1, R2, R3, R4, R'4, R5, R6, R9, R10,** et les coefficients n et n' ont les mêmes significations générales et plus particulières indiquées auparavant.

De préférence, les radicaux :
**R1, R2, R3,** identiques ou différents, représentent un radical alkyle linéaire ou ramifié en C1-C4,
**R4, R'4,** représentent un atome d'hydrogène,
**R5** représente un atome d'hydrogène, un groupement nitro ;
**R6,** représente un atome d'hydrogène, un radical alkyle linéaire ou ramifié en C1-C4, de préférence méthyle,
**R9, R10,** identiques ou non, représentent un atome d'hydrogène, un atome de chlore, un atome de fluor, un radical hydroxyle, un radical amino, un radical amino substitué par un ou deux radicaux, identiques ou différents, linéaires ou ramifiés, alkyle en C1-C4, hydroxyalyle en C1-C4, un cycle pyrrolidine éventuellement substitué par un radical amino substitué par un ou plusieurs radicaux, identiques ou différents, alkyle en C1-C4 éventuellement porteurs d'au moins un groupement hydroxyle, de préférence méthyle, un radical trialkyl(C1-C4)ammonium avec des radicaux alkyle, identiques ou non, linéaires ou ramifiés, de préférence méthyle
**R9** et **R10,** identiques ou non, ne représentant pas simultanément un atome de chlore ou un atome de fluor ;
l'électroneutralité des composés de formule (III) ou de ses sels, étant assurée par un ou plusieurs contre ions **An,** identiques ou non, cosmétiquement acceptables, choisis notamment parmi ceux listés auparavant.

Il est en outre précisé que selon un mode de réalisation particulier, le groupement ammonium se trouve en position 7 sur le noyau naphtalène.

Conformément à une deuxième variante particulière de l'invention, les composés correspondent à la formule suivante (IVa) ou ses sels :

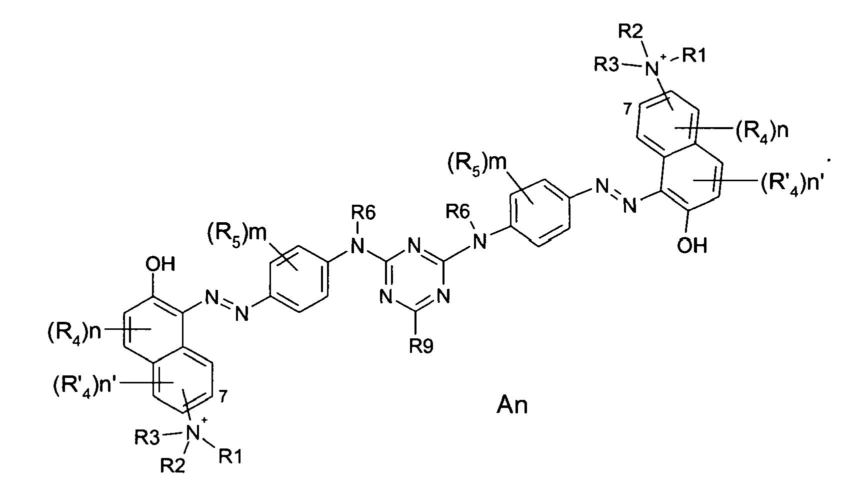

Formule dans laquelle les radicaux **R1, R2, R3, R4, R'4, R5, R6, R9,** et les coefficients n et n'ont les mêmes significations générales et plus particulières indiquées auparavant.

Conformément à cette variante particulière de l'invention, les composés correspondent à la formule suivante (IVb) :

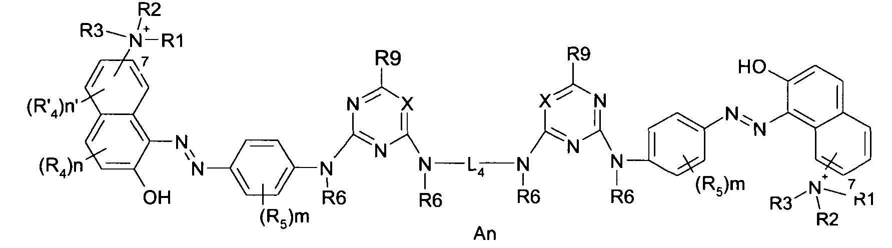

Formule dans laquelle les radicaux **R1, R2, R3, R4, R'4, R5, R6, R9,** et les coefficients n et n' ont les mêmes significations générales et plus particulières indiquées auparavant.

De préférence, dans les formules (IVa) et (IVb), les radicaux :
**R1, R2, R3,** identiques ou différents, représente un radical alkyle linéaire ou ramifié en **C1-C4,**
**R4, R'4,** représentent un atome d'hydrogène,
**R5** représente un atome d'hydrogène, un groupement nitro ;
**R6,** représente un atome d'hydrogène, un radical alkyle linéaire ou ramifié en C1-C4, de préférence méthyle,
**R9** représente un atome d'hydrogène, un atome de chlore, un atome de fluor, un radical hydroxyle, un radical amino, un radical amino substitué par un ou deux radicaux, identiques ou différents, linéaires ou ramifiés, alkyle en C1-C4, hydroxyalyle en C1-C4, un cycle pyrrolidine éventuellement substitué par un radical trialkylammonium avec des radicaux alkyle, identiques ou non, linéaires ou ramifiés, en C1-C4, de préférence méthyle ;
l'électroneutralité des composés de formule (IV) ou de leurs sels, étant assurée par un ou plusieurs contre ions **An** cosmétiquement acceptables, choisis notamment parmi ceux listés auparavant.

Il est en outre précisé que selon un mode de réalisation particulier, le groupement ammonium des formules (IVa) et (IVb) se trouve en position 7 sur le noyau naphtalène.

Conformément à une troisième variante particulière de l'invention, les composés correspondent à la formule suivante (V) :

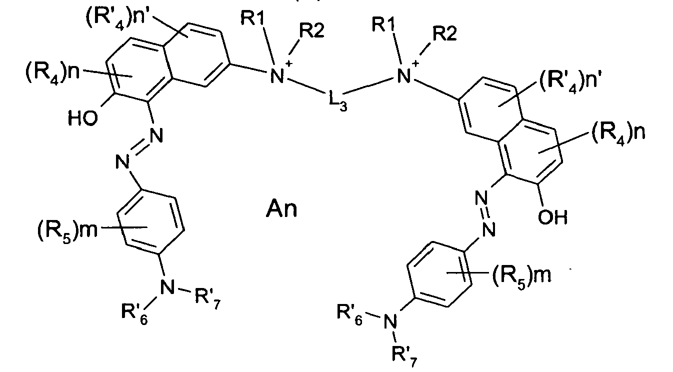

Formule dans laquelle les radicaux **R4, R'4, R5, R'6, R'7, R1, R2,** et les coefficients n et n'ont les mêmes significations générales et plus particulières indiquées auparavant.

De préférence, les radicaux :
**R4, R'4,** représentent un atome d'hydrogène ;
**R5** représente un atome d'hydrogène, un groupement nitro ;
**R'6, R'7,** représentent un atome d'hydrogène ;
**R1, R2**, identiques ou différents, représentent un radical alkyle, linéaire ou ramifié, en C1-C4, un radical hydroxyalkyle linéaire ou ramifié, en C1-C4 ;
**L3** représente un radical alkylène, linéaire ou ramifié, comprenant de 1 à 10 atomes de carbone, et de préférence de 2 à 8 atomes de carbone ;
l'électroneutralité des composés de formule (V) ou de leurs sels, étant assurée par un ou plusieurs contre ions **An** cosmétiquement acceptables, choisis notamment parmi ceux listés auparavant.

Des procédés de préparation des composés de formules (I), (II) et leurs variantes particulières (III) à (V) vont maintenant être décrits.

Ainsi, un premier procédé de préparation de composés de formule (I) et notamment de formule (III), consiste à mettre en contact au moins un composé de formule (VI) : 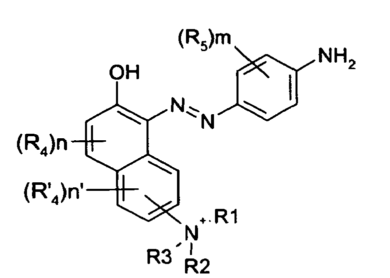 avec un halogénure cyanurique, en présence d'un solvant ou d'un mélange de solvant pour obtenir un composé de formule (I) ou ses sels, dans lequel R7 représente un groupe de formule (a).

Généralement, le composé de formule (VI) est mis en oeuvre sous une forme solubilisée dans un solvant approprié.

De manière avantageuse, le solvant peut être de l'eau ou un mélange d'eau avec un ou plusieurs solvants organiques. De préférence, les solvants organiques sont choisis parmi les solvants miscibles solubles à l'eau, comme par exemple l'éthanol, les glycols, l'alcool benzylique, l'acétone, ou leurs mélanges.

La réaction peut avoir lieu en présence d'au moins un additif tel qu'un agent de contrôle du pH. De préférence le pH du milieu réactionnel varie de 3 à 6.

Cet agent de contrôle du pH est habituellement choisi parmi, à titre d'exemples illustratifs, l'ammoniaque, les carbonates alcalins, les hydrogénocarbonates alcalins, les alcanolamines telles que les mono-, di- et triéthanolamines ainsi que leurs dérivés, les hydroxydes de sodium ou de potassium et les composés de formule (A) suivante : 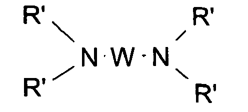 dans laquelle W est un reste propylène éventuellement substitué par un groupement hydroxyle ou un radical alkyle en C₁-C₆ ; les radicaux R', identiques ou différents, représentent un atome d'hydrogène, un radical alkyle en C₁-C₆ ou hydroxyalkyle en C₁-C₆.

On peut aussi utiliser si nécessaire, des acides minéraux ou organiques comme l'acide chlorhydrique, l'acide orthophosphorique, l'acide sulfurique, l'acide acétique.

Avantageusement, le pH est maintenu dans une gamme variant de 3 à 6 par addition d'un agent de contrôle du pH durant la réaction.

Il peut être préférable de maintenir pendant la durée de l'introduction du composé (VI) dans les composés à fonction cyano, la température du mélange réactionnel à une température inférieure à la température ambiante, et de préférence comprise entre 0 et 10°C.

Une fois l'introduction des réactifs terminée, le mélange réactionnel est maintenu à une température supérieure ou égale à la température ambiante.

La durée de la réaction est habituellement comprise entre 1 et 10 heures.

On récupère le produit en mettant en oeuvre les moyens classiques, comme par exemple l'évaporation du solvant, la filtration.

Pour l'obtention de composés dans lesquels R9 ne représente pas un atome d'halogène, la réaction de substitution de l'atome d'halogène par le radical souhaité peut être faite de manière classique pour l'homme du métier.

Un deuxième procédé de préparation de composés de formule (1) dans laquelle R7 représente un radical de formule (b) consiste à mettre en contact au moins un composé de formule (VI) 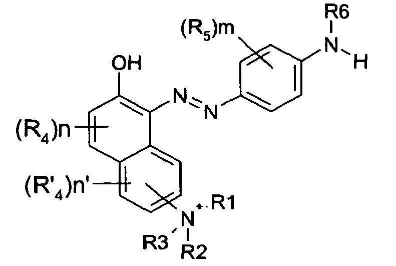 avec un composé précurseur de L2 ou avec un composé comprenant L2 et deux fonctions susceptibles de réagir avec les fonctions amine du composé (VI), en présence d'un solvant ou d'un mélange de solvant.

Une première variante consiste à mettre en oeuvre le procédé décrit ci-dessus pour obtenir un composé de formule (I) dans lequel R7 représente un groupe de formule (a), avec les quantités appropriées en composé de formule (VI) et d'halogénure cyanurique.

Il n'est pas nécessaire de manière avantageuse, d'isoler le produit intermédiaire obtenu.

Là encore, dans le cas de composés dans lesquels R10 ne représente pas un atome d'halogène, la réaction de substitution de l'atome d'halogène par le radical souhaité peut être faite de manière classique pour l'homme du métier. On peut par exemple mettre en contact ledit produit avec une amine, de l'eau, en chauffant, dans un solvant approprié.

Selon une deuxième variante, on met en contact un composé de formule (VII) suivante : 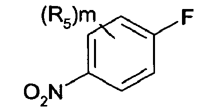 avec une diamine de formule R₆NH-L₂-NHR₆ pour obtenir un composé de formule (VIII) suivante :

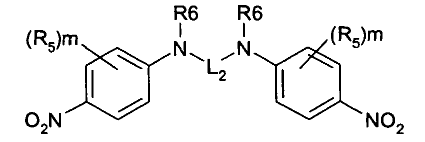

Cette étape est réalisée de manière classique dans le domaine.

Elle a lieu en général en présence d'un solvant. Parmi les solvants convenables, on peut citer par exemple le toluène, le dichlorométhane, le méthanol, l'éthanol, ou leurs mélanges.

La température à laquelle la réaction est mise en oeuvre est habituellement comprise entre 5 et 80°C.

On effectue ensuite une étape de réduction des groupements des groupements nitro du composé ainsi obtenu, suivie d'une étape de diazotation. On obtient alors un composé de formule (VIII) suivante :

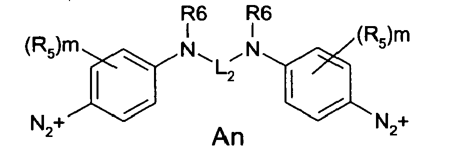

La réduction des groupements nitro est réalisée de manière habituelle, par exemple sous pression d'hydrogène en présence d'un catalyseur tel que par exemple le palladium.

Classiquement, la pression d'hydrogène est comprise entre 0,01 et 10 bar.

La température de réaction est notamment comprise entre 5 et 80°C.

La réduction est réalisée oeuvre dans un solvant approprié. Classiquement, les solvants organiques sont choisis par exemple parmi le toluène, le dichlorométhane, le méthanol, l'éthanol, ou leurs mélanges.

L'étape de diazotation consiste à mettre en contact le produit réduit avec du nitrite de sodium (NaNO2)

La température est en général comprise entre 0 et 10°C.

A l'issue de cette étape, le produit obtenu est mis en contact avec un composé de formules (IXa) ou (IXb) suivantes :

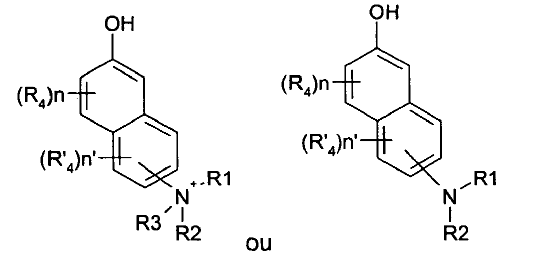

Cette étape a lieu habituellement à une température comprise entre 0 et 10°C.

Par ailleurs, le pH auquel la réaction est réalisé est habituellement compris entre 6 et 10.

Le solvant de la réaction est avantageusement l'eau.

Au cas où l'on utilise le composé comprenant la fonction amine (IXb), on effectue une étape supplémentaire, classique, de quaternisation du produit issu de l'étape précédente.

Le produit est récupéré de manière classique, par exemple par évaporation du solvant, par filtration.

Il est précisé en outre qu'il n'est pas nécessaire d'isoler le produit obtenu à chaque étape avant de l'engager dans une étape suivante, à moins d'une incompatibilité (par exemple solvants différents).

Les composés de formule (II) peuvent être obtenus en mettant en oeuvre les étapes suivantes :

On met en contact un composé de formule (X) suivante : 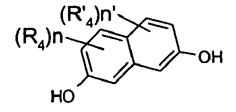 avec une diamine de formule H₂N-L₃-NH₂ et l'on obtient un composé de formule suivante (XI) :

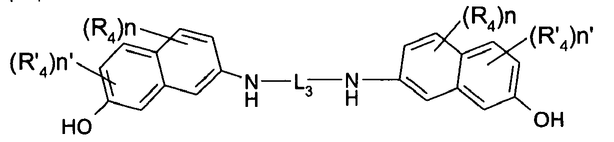

Cette réaction est habituellement mise en oeuvre en présence d'un solvant ou d'un mélange de solvants. A titre de solvant approprié, on peut citer par exemple les alcools, le toluène, le dichlorométhane, l'eau, ainsi que leurs mélanges.

La température à laquelle cette étape est mise en oeuvre varie en général de 0 à 110°C.

Le composé est alors N-alkylé puis quaternisé de manière classique.

On fait ensuite réagir le produit ainsi obtenu avec un composé de formule (XII) suivante :

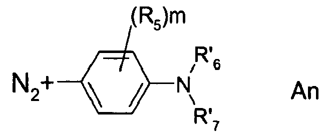

Ce composé est obtenu de manière habituelle par diazotation de la diamine aromatique correspondante. Les conditions de mise en oeuvre de cette étape sont similaires à celles rappelées dans le procédé précédemment détaillé.

Le produit est récupéré de manière classique, par exemple par évaporation du solvant, par filtration.

Un autre objet de la présente invention est constitué par une composition tinctoriale comprenant, dans un milieu approprié pour la teinture des fibres kératiniques, au moins un colorant direct choisi parmi les composés de formules (I) et (II) ou leurs sels d'addition, et qui viennent d'être détaillés.

De manière avantageuse, la teneur en chacun des composés de formules (I) et (II) ou leurs sels, varie de 0,001 à 20 % en poids par rapport au poids de la composition tinctoriale. De préférence, la teneur en chacun de ces composés varie de 0,01 à 10 % en poids par rapport au poids de la composition tinctoriale.

La composition tinctoriale selon l'invention peut contenir un ou plusieurs colorants directs additionnels différents des composés de formules (I) et (II).

On peut utiliser les colorants directs classiquement mis en oeuvre dans le domaine de la coloration des fibres kératiniques, et notamment des fibres kératiniques humaines.

A ce titre, on peut notamment citer les colorants nitrés de la série benzénique, les colorants additionnels directs azoïques, les colorants directs méthiniques. Ces colorants directs peuvent être de nature non ionique, anionique ou cationique. De préférence ces colorants directs additionnels sont de nature cationique.

Selon un mode de réalisation particulier de l'invention, la composition tinctoriale comprend une teneur en chacun des colorants directs variant de 0,001 à 10 % en poids par rapport au poids total de la composition tinctoriale.

La composition tinctoriale de l'invention peut aussi contenir une ou plusieurs bases d'oxydation et/ou un ou plusieurs coupleurs, conventionnellement utilisés pour la teinture de fibres kératiniques, et notamment des fibres kératiniques humaines.

Parmi les bases d'oxydation, on peut citer les para-phénylènediamines, les bis-phénylalkylènediamines, les para-aminophénols, les bis-para-aminophénols, les ortho-aminophénols, les bases hétérocycliques et leurs sels d'addition.

La ou les bases d'oxydation présentes dans la composition de l'invention sont en général utilisées chacune en quantité variant de 0,001 à 10 % en poids par rapport au poids total de la composition tinctoriale, de préférence variant de 0,005 à 6 % en poids.

Parmi les coupleurs utilisables, on peut notamment citer les méta-phénylènediamines, les méta-aminophénols, les méta-diphénols, les coupleurs naphtaléniques, les coupleurs hétérocycliques et leurs sels d'addition.

Dans la composition de la présente invention, le ou les coupleurs sont chacun généralement présents en quantité variant de 0,001 à 10 % en poids par rapport au poids total de la composition tinctoriale, de préférence variant de 0,005 à 6 % en poids.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

Le milieu approprié pour la teinture, appelé aussi support de teinture, est un milieu cosmétique généralement constitué par de l'eau ou par un mélange d'eau et d'au moins un solvant organique pour solubiliser les composés qui ne seraient pas suffisamment solubles dans l'eau.

A titre de solvant organique, on peut par exemple citer les monoalcools, linéaires ou ramifiés, de préférence saturés, comprenant 2 à 10 atomes de carbone, tels que l'alcool éthylique, l'alcool isopropylique ; les alcools aromatiques tels que l'alcool benzylique, l'alcool phényléthylique ; les polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol, l'hexylèneglycol (2-méthyl 2,4-pentanediol), le néopentylglycol et le 3-méthyl-1,5-pentanediol ; ainsi que les alkyléthers de diéthylèneglycol, notamment en C₁-C₄, comme par exemple, le monoéthyléther ou le monobutyléther du diéthylèneglycol, seuls ou en mélange.

Les solvants sont, de préférence, présents dans des proportions allant de 1 à 40 % en poids environ par rapport au poids total de la composition tinctoriale, et encore plus préférentiellement de 5 à 30 % en poids environ.

La composition tinctoriale conforme à l'invention peut également renfermer divers adjuvants utilisés classiquement dans les compositions pour la teinture des fibres kératiniques, notamment humaines et en particulier les cheveux, tels que des agents tensioactifs anioniques, cationiques, non-ioniques, amphotères, zwitterioniques ou leurs mélanges ; des polymères anioniques, cationiques, non ioniques, amphotères, zwitterioniques ou leurs mélanges ; des agents épaississants minéraux ou organiques, et en particulier les épaississants associatifs polymériques anioniques, cationiques, non ioniques et amphotères ; des agents antioxydants ; des agents de pénétration ; des agents séquestrants ; des parfums ; des tampons ; des agents dispersants ; des agents de conditionnement tels que par exemple des silicones volatiles ou non volatiles, modifiées ou non modifiées ; des agents filmogènes ; des céramides ou pseudo-céramides ; des agents conservateurs ; des agents opacifiants, etc.

Les adjuvants ci dessus sont en général présents en quantité variant, pour chacun d'eux, de 0,01 à 20 % en poids par rapport au poids de la composition.

La composition de l'invention peut en outre contenir au moins un agent oxydant.

Les agents oxydants classiquement utilisés pour la teinture d'oxydation des fibres kératiniques, notamment humaines, sont par exemple le peroxyde d'hydrogène, le peroxyde d'urée, les bromates ou ferricyanures de métaux alcalins, les persels tels que les perborates et les persulfates de métaux alcalins ou alcalino-terreux, comme le sodium, le potassium, le magnésium, seuls ou en mélanges, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.
La composition de l'invention peut de plus comprendre au moins un agent alcalin qui peut être choisi parmi ceux utilisés classiquement dans le domaine cosmétique. On pourra se reporter à la liste de ces composés indiquée auparavant.

Le pH de la composition tinctoriale de l'invention, c'est-à-dire de la composition dépourvue d'agent oxydant, est de préférence compris entre 5 et 12, de préférence entre 7 et 11.

Bien entendu, l'homme de l'art veillera à choisir ce ou ces éventuels composés complémentaires de manière telle que les propriétés avantageuses attachées intrinsèquement à la composition selon l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, notamment humaines, et plus particulièrement des cheveux.

Le procédé de la présente invention est un procédé dans lequel on applique sur les fibres, sèches ou non, la composition selon la présente invention telle que définie précédemment.

Selon une première variante, la composition appliquée sur les fibres kératiniques, ne comprend pas d'agent oxydant. Cette variante est particulièrement appropriée lorsque la composition tinctoriale comprend au moins un colorant selon l'invention et éventuellement au moins un colorant direct additionnel.

Selon une deuxième variante de l'invention, le procédé est mis en oeuvre avec au moins un agent oxydant. Cette deuxième variante est appropriée quelle que soit la nature des colorants présents (colorant selon l'invention, colorant direct additionnel, base d'oxydation et/ou coupleur).

Selon cette deuxième variante, l'agent oxydant peut être ajouté à la composition tinctoriale au moment de l'emploi ou bien il peut encore être mis en oeuvre à partir d'une composition oxydante le comprenant, appliquée simultanément ou séquentiellement à la composition tinctoriale comprenant le colorant. Dans ce dernier cas, l'agent oxydant est présent dans une composition différente de celle comprenant le colorant.

Selon un mode de réalisation particulier, la composition comprenant le colorant est mélangée, de préférence au moment de l'emploi, à une composition contenant, dans un milieu approprié pour la teinture, au moins un agent oxydant, cet agent oxydant étant présent en une quantité suffisante pour obtenir l'éclaircissement souhaité.

Le mélange obtenu est ensuite appliqué sur les fibres kératiniques.

Après un temps de pose suffisant pour obtenir la coloration désirée, habituellement variant de 3 minuutes à 1 heure environ, de préférence 5 à 40 minutes environ, les fibres kératiniques sont de préférence rincées, puis éventuellement lavées au shampooing, rincées à nouveau puis séchées ou laissées sécher.

Par ailleurs, de manière classique la composition est appliquée et laissée agir à une température allant de 15 à 80°C, de préférence de 15 à 40°C.

La composition oxydante peut également renfermer divers adjuvants utilisés classiquement dans les compositions pour la teinture des fibres kératiniques, notamment humaines, et tels que définis précédemment.

Le pH de la composition oxydante renfermant l'agent oxydant est tel qu'après mélange avec la composition tinctoriale, le pH de la composition résultante appliquée sur les fibres kératiniques (soit en d'autres termes la composition prête à l'emploi) varie de préférence de 7 à 12 environ, et encore plus préférentiellement de 7 à 11. Il peut être ajusté à la valeur désirée au moyen d'agent(s) acidifiant(s) ou alcalinisant(s).

Parmi les agents acidifiants, on peut citer, à titre d'exemples, les acides minéraux ou organiques comme l'acide chlorhydrique, l'acide orthophosphorique, l'acide sulfurique, l'acide acétique.

La composition prête à l'emploi, soit en d'autres termes la composition qui est finalement appliquée sur les fibres kératiniques, peut se présenter sous des formes diverses, telles que sous forme de liquides, de crèmes, de gels ou sous toute autre forme appropriée pour réaliser une teinture des fibres kératiniques, notamment des fibres kératiniques humaines, telles que les cheveux.

L'invention a aussi pour objet un dispositif à plusieurs compartiments dans lequel au moins un premier compartiment comprend une composition tinctoriale comprenant au moins un colorant tel que décrit auparavant, et éventuellement au moins un colorant direct différent du colorant, éventuellement au moins une base d'oxydation, éventuellement au moins un coupleur, et un autre compartiment comprend un agent oxydant.

Il est à noter que le ou les colorants, éventuellement le colorant direct additionnel, la ou les bases d'oxydation, le ou les coupleurs, peuvent se trouver dans un même compartiment ou dans plusieurs ; un même compartiment pouvant comprendre un seul type de colorant (mixte, direct additionnel, oxydation) ou une combinaison de plusieurs d'entre eux.

Ce dispositif peut être équipé d'un moyen permettant de délivrer sur les fibres à traiter, le mélange souhaité, tel que les dispositifs décrits dans le brevet FR 2 586 913

Les exemples suivants servent à illustrer l'invention sans toutefois en limiter la portée.

### EXEMPLES

### A - Synthèse de colorants :

### EXEMPLE 1

### Synthèse du composé 1 : dichlorure de 8-{(E)-[4-({4-chloro-6-[(4-{(E)-[2-hydroxy-7-(trimethylammonio)-1-naphthyl]diazenyl}phenyl)amino]-1,3,5-triazin-2-yl}amino)phenyl]diazenyl}-7-hydroxy-N,N,N-trimethylnaphthalen-2-aminium.

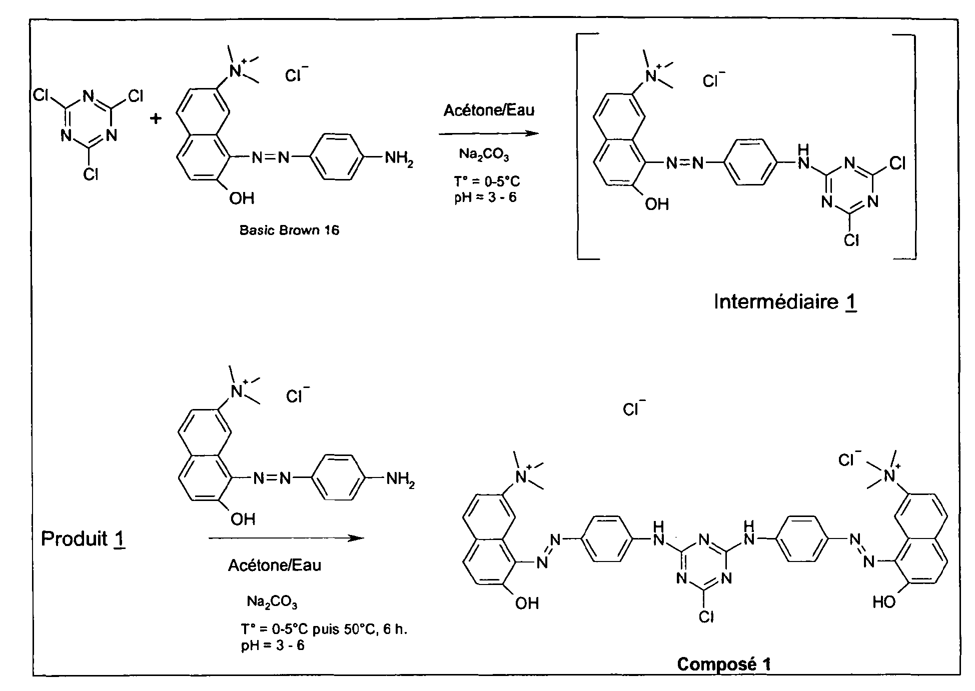

On dissout le colorant Basic Brown 16 (20g) dans 200 ml d'eau. La solution obtenue est alors introduite lentement dans une solution comprenant du chlorure cyanurique (0,5 mole eq), de l'acétone (30ml) et de la glace (50g).

Pendant l'addition, on maintient la température entre 0 et 5°C en utilisant un bain d'eau et de glace. Le pH est maintenu entre 3 et 6 par ajout de bicarbonate de sodium solide.

Après l'introduction, le mélange réactionnel est maintenu sous agitation à 40°C pendant une heure puis pendant 6 heures à 50°C.

La stabilisation du pH indique que la réaction est terminée.

Le mélange réactionnel est ensuite maintenu sous agitation à température ambiante pendant 16 heures et l'on obtient un fin précipité qui est filtré et séché sous vide.

L'analyse du spectre RMN du proton et spectrographie de masse confirme l'obtention du produit attendu.

### EXEMPLES 2 et 3

### Synthèse du composé 2 : diformate de 7-hydroxy-8-{(E)-[4-({4-[(2-hydroxyethyl)amino]-6-[(4-{(E)-[2-hydroxy-7-(trimethylammonio)-1-naphthyl]diazenyl}phenyl)amino]-1,3,5-triazin-2-yl}amino)phenyl]diazenyl}-N,N,N-trimethylnaphthalen-2-aminium.

### Synthèse du composé 3 : diformate de 7-hydroxy-8-{(E)-[4-({4-hydroxy-6-[(4-{(E)-[2-hydroxy-7-(trimethylammonio)-1-naphthyl]diazenyl}phenyl)amino]-1,3,5-triazin-2-yl}amino)phenyl]diazenyl}-N,N,N-trimethylnaphthalen-2-aminium.

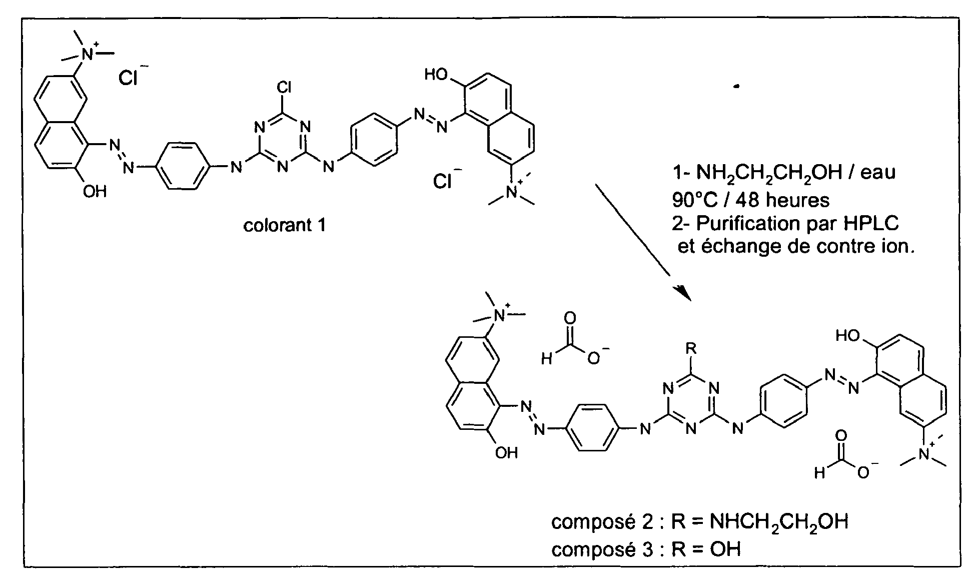

On dissout le composé **1** précédemment obtenu (1g) dans 100 ml d'eau et on ajoute la monoéthanolamine (1g).

Après l'introduction, le mélange réactionnel est maintenu sous agitation à 90°C pendant 48 heures puis évaporé à sec.

Un échantillon du mélange réactionnel est purifié par chromatographie avec simultanément changement de contre ion (CI- en CH₃COO-).

On obtient alors un colorant 2 dans lequel R représente le groupement NHCH₂CH₂OH, et un colorant 3 dans lequel R représente un groupement hydroxyle.

### EXEMPLE 4

### Synthèse du composé 4 : le chlorure de 8-((E)-{4-[(4,6-dipyrrolidin-1-yl-1,3,5-triazin-2-yl)amino]phenyl}diazenyl)-7-hydroxy-N,N,N-trimethylnaphthalen-2-aminium chloride

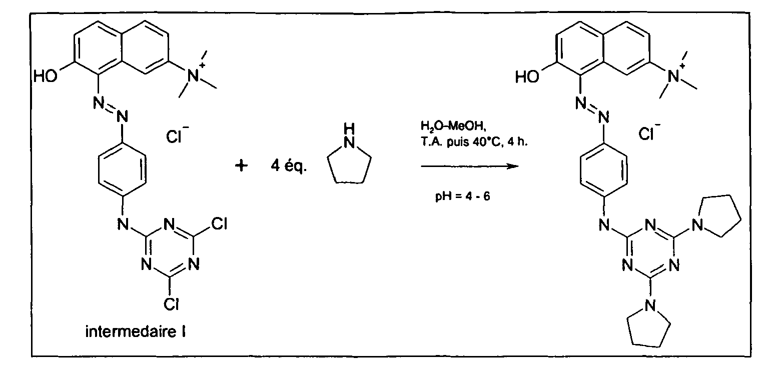

Le composé intermédiaire I est obtenu de la manière suivante :

Comme dans le cas de l'exemple 1, on dissout le colorant Basic Brown 16 (20g) dans 200 ml d'eau. La solution obtenue est alors introduite lentement dans une solution comprenant du chlorure cyanurique (0,5 mole eq), de l'acétone (30ml) et de la glace (50g).

Pendant l'addition, on maintient la température entre 0 et 5°C en utilisant un bain d'eau et de glace. Le pH est maintenu entre 3 et 6 par ajout de bicarbonate de sodium solide.

La stabilisation du pH indique que la réaction est terminée.

Le produit est alors précipité à l'aide d'acétone puis filtré et enfin séché au dessicateur sous vide.

L'analyse du spectre RMN du proton et spectrographie de masse confirme l'obtention du composé intermédiaire I.

On solubilise le composé intermédaire I (1g) dans 8 ml d'un mélange constitué d'eau et de méthanol (1/1).

On coule goutte à goutte 4 équivalents de pyrrolidine en maintenant le pH à une valeur comprise entre 4 et 6 à l'aide d'une solution d'acide chlorhydrique 1 N. Après ajout de la pyrrolidine, le milieu réactionnel est laissé sous agitation 12 heures puis chauffé pendant 2 heures à 40°C.

Par la suite, le milieu réactionnel est versé dans 50 ml d'acétone puis filtré. La poudre obtenue est lavée 3 fois avec 50ml d'acétone, puis 3 fois avec 50ml d'éther diisopropylique, et séchée au dessicateur sous vide

L'analyse du spectre RMN du proton et spectrographie de masse confirme l'obtention du produit attendu.

### EXEMPLE 5

### Synthèse du composé 5: le chlorure de 8-{(E)-[4-({4,6-bis[3-(dimethylamino)pyrrolidin-1-yl]-1,3,5-triazin-2-yl}amino)phenyl]diazenyl)-7-hydroxy-N,N,N-trimethylnaphthalen-2-aminium

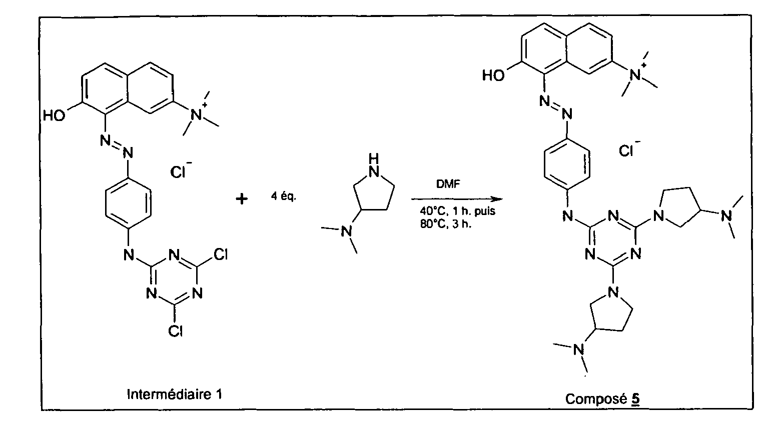

On solubilise le composé intermédaire I (1g) dans 10 ml de diméthylformamide à 40°C.

On coule 2 équivalents de N,N-dimethylpyrrolidin-3-amine , dilués dans 2 ml de DMF, et on maintient le milieu réactionnel pendant 1 heure à 40°C, puis on chauffe à 80°C, pendant 2 heures.

On coule de nouveau 2 équivalents de N,N-dimethylpyrrolidin-3-amine, dilués dans 2 ml de DMF et le milieu réactionnel est maintenu 1 heure à 80°C.

Par la suite, on verse le mélange réactionnel dans 50 ml d'acétone puis le précipité obtenu est filtré.

La poudre obtenue est lavée 3 fois avec 50 ml d'acétone, puis 3 fois avec 50 ml d'éther diisopropylique. Le produit est séché au dessicateur sous vide ;

L'analyse du spectre RMN du proton et spectrographie de masse confirme l'obtention du produit attendu.

### EXEMPLE 6

### Synthèse du composé 6: chlorure / bisméthylsulfate de 1-{4-[(4-{(E)-[2-hydroxy-7-(trimethylammonio)-1-naphthyl]diazenyl}phenyl)amino]-6-[3-(trimethylammonio)pyrrolidin-1-yl]-1,3,5-triazin-2-yl}-N,N,N-trimethylpyrrolidin-3-aminium

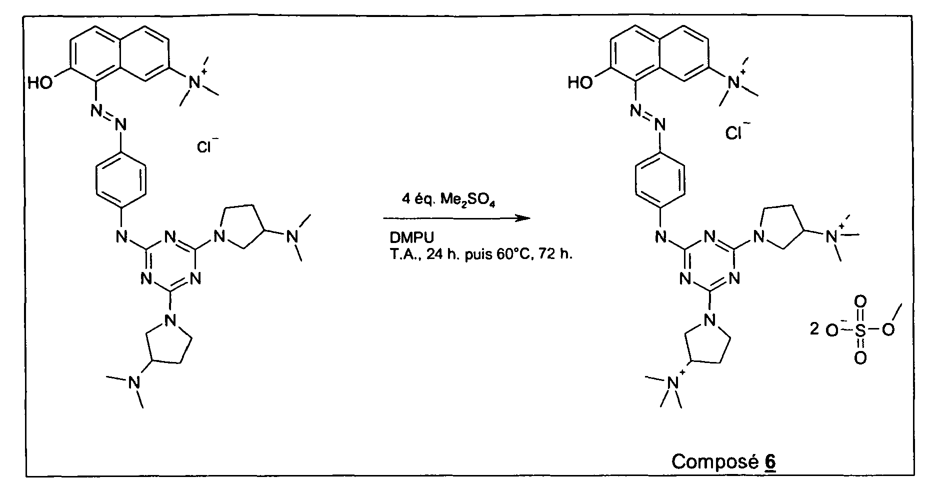

On solubilise le composé **5** précédemment obtenu (0,1g) dans 2 ml de 1,3-diméthyl-3,4,5,6-tétrahydro-2(1H)-pyrimidinone (DMPU).

On coule 2 équivalents de diméthylsulfate, et on laisse le milieu réactionnel sous agitation à température ambiante pendant 24 heures.

On ajoute 2 autres équivalents de diméthylsulfate ; le milieu réactionnel est porté à 60°C pendant 72 h.

Par la suite, le mélange réactionnel est versé dans 30 ml d'éther diisopropylique puis filtré. La poudre obtenue est lavée 3 fois avec 50 ml d'éther diisopropylique.

L'analyse du spectre RMN du proton et spectrographie de masse confirme l'obtention du produit attendu.

### B - Exemples de coloration

On prépare une solution tamponnée à pH 9 par mélange de 2 g d'acétate d'ammonium dans 30 ml d'eau et 10ml alcool benzylique puis par ajustement du pH par addition d'ammoniaque et le volume est complété à 100 ml par addition d'eau désionisée.

On prépare autant d'échantillons que de composés obtenus aux exemples précédents, chaque échantillon comprenant un seul colorant, avec une concentration de 5.10⁻⁴ mol pour 100 g en colorant dans la solution tamponnée précitée.

On met en contact, pour chaque échantillon, une mèche de cheveux blancs sensibilisés avec la solution résultante, avec un rapport de bain de 1 pour 10.

Après 30 minutes de pose, chaque mèche est rincée à l'eau désionisée pour éliminer l'excès de solution de colorant.

On obtient pour chacun des colorants, une mèche de cheveux colorée en brun.

### Test Comparatif :

On prépare deux échantillons, le premier comprenant le colorant Basic Brown 16, et le second comprenant le colorant 1 obtenu à l'exemple 1, chaque échantillon comprenant 5.10⁻⁴ mol % de colorant, dans la solution tamponnée décrite ci-dessus.

On met en contact, pour chaque échantillon, une mèche de cheveux blancs sensibilisés avec la solution résultante, avec un rapport de bain de 1 pour 10.

Après 30 minutes de pose, chaque mèche est rincée à l'eau désionisée pour éliminer l'excès de solution de colorant.

### Test de shampooing

Chaque mèche de cheveux colorée selon l'étape précédente est lavée à la main avec une solution comprenant 1 % en volume de shampooing Mixa Bébé, pendant 30 secondes, puis rincée avec 200 ml d'eau. Le procédé est répété 10 fois.

### Résultats du test de shampooing

Les mèches obtenues dans les deux cas ont conservé la même couleur mais l'intensité de la couleur de la mèche colorée avec le colorant 1 conforme à l'invention est visuellement plus importante que celle de la mèche colorée avec le colorant Basic Brown 16 n'entrant pas dans le cadre de l'invention.

Cela montre par conséquent, que la composition tinctoriale selon l'invention permet d'obtenir des colorations plus résistantes aux shampooings.

## Revendications

1. Composés de formules (I) et (II) suivantes, et leurs sels d'addition : 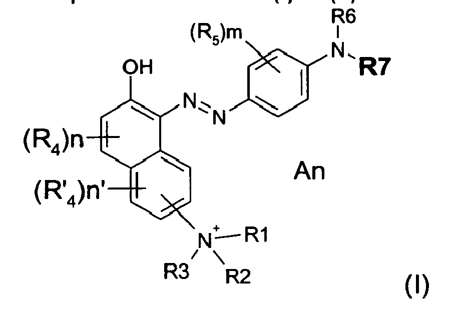 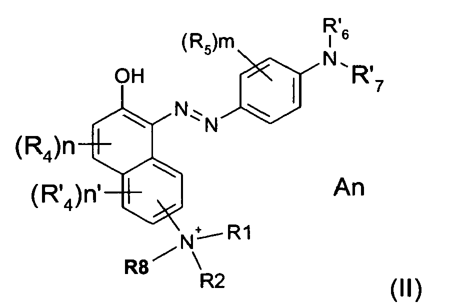 Formules dans lesquelles :
**R1, R2, R3,** identiques ou différents, représentent :
• un radical alkyle en C1-C4, linéaire ou ramifié, substitué ou non par au moins :
o un groupement hydroxyle ;
o un groupement alcoxy, linéaire ou ramifié, en C1-C4 ;
o un groupement amino ;
o un groupement amino substitué par un ou deux radicaux, identiques ou différents, alkyle en C1-C4 linéaires ou ramifiés, hydroxyalkyle en C1-C4 linéaires ou ramifiés, deux des radicaux **R1, R2** ou **R3** pouvant éventuellement former un hétérocycle à 5 ou 6 chaînons, saturé ou insaturé, comprenant éventuellement un autre hétéroatome choisi parmi l'oxygène, l'azote, et éventuellement substitué ;
**R4, R'4, R5,** identiques ou différents, représentent :
• un atome d'halogène, de préférence le chlore, le fluor ;
• un groupement hydroxyle ;
• un groupement nitro, cyano ;
• un radical alkyle, linéaire ou ramifié, en C1-C4, éventuellement porteur d'au moins un groupement hydroxyle, au moins un groupement alcoxy linéaire ou ramifié en C1-C4, au moins un groupement amino et/ou au moins un groupement amino substitué par un ou deux radicaux alkyle, identiques ou non, linéaires ou ramifiés en C1-C4 ;
• un radical alcoxy, linéaire ou ramifié, en C1-C4 ;
• un radical amino ;
• un radical amino substitué par un ou deux radicaux, identiques ou différents, choisis parmi les radicaux alkyle en C1-C4, linéaires ou ramifiés, éventuellement substitués par un ou deux radicaux choisis parmi :
■ un groupement hydroxyle,
■ un groupement amino,
■ un groupement amino substitué par un ou deux radicaux, identiques ou différents, linéaires ou ramifiés, alkyle en C1-C4, hydroxyalkyle en C1-C4 ;
■ un groupement amino substitué dont les radicaux forment un hétérocycle à 5 ou 6 chaînons, saturé ou insaturé, comprenant éventuellement autre hétéroatome choisi parmi l'oxygène, l'azote ; ledit hétérocycle étant éventuellement substitué ;
• un groupement -SO₂NHR dans lequel R représentant un atome d'hydrogène, un radical alkyle en C1-C4, éventuellement substitués par un ou plusieurs radicaux hydroxyle, amino, amino substitué par un ou deux radicaux, identiques ou différents, alkyle en C1-C4 linéaires ou ramifiés, hydroxyalkyle en C1-C4 linéaires ou ramifiés
deux radicaux **R5** portés par deux atomes de carbone adjacents peuvent également former avec l'atome de carbone auquel chacun est rattaché, un cycle aromatique à 6 chaînons éventuellement substitué ;
R6, R'6, R'7, identiques ou différents, représentent :
■ un atome d'hydrogène,
■ un radical alkyle, linéaire ou ramifié en C1-C8, substitué ou non par un groupement hydroxyle, alcoxy linéaire ou ramifié en C1-C4, amino, amino substitué par un ou deux radicaux, identiques ou différents, alkyle linéaire ou ramifié en C1-C4, hydroxyalkyle linéaire ou ramifié en C1-C4 ; interrompu ou non par un hétéroatome choisi parmi l'oxygène, l'azote ou par un groupe comprenant un hétéroatome tel que de préférence CO, SO₂ ;
■ les radicaux R'6 et R'7 pouvant éventuellement former un hétérocycle à 5 ou 6 chaînons, saturé ou insaturé, comprenant éventuellement autre hétéroatome choisi parmi l'oxygène, l'azote ; ledit hétérocycle étant éventuellement substitué ;
**R7** représente un groupement choisi parmi : 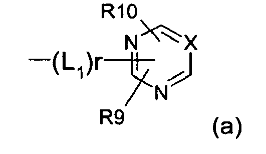 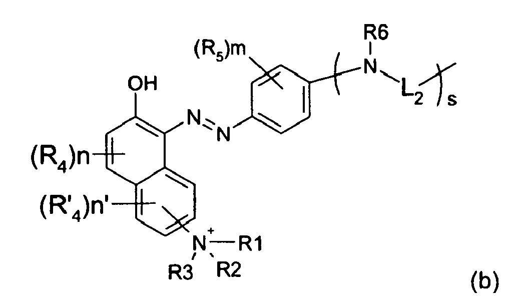
**R8** représente le groupement suivant : 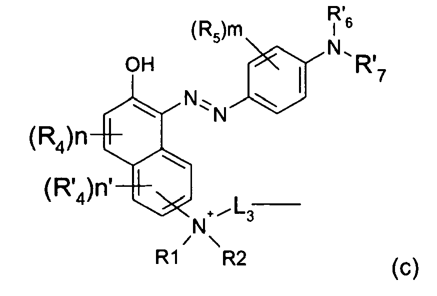
**R1, R2, R3, R4, R'4, R5, R6, R'6, R'7,** dans les formules (a), (b) et (c) ayant la même signification que précédemment ;
**R9, R10**, identiques ou non, représentent :
• un atome d'hydrogène,
• un atome d'halogène, de préférence le chlore, le fluor,
• un groupement nitro, cyano,
• un radical alkyle, linéaire ou ramifié, en C1-C4, éventuellement porteur d'au moins un groupement hydroxyle, au moins un groupement alcoxy linéaire ou ramifié en C1-C4, au moins un groupement amino et/ou au moins un groupement amino substitué par un ou deux radicaux alkyles, identiques ou non, linéaires ou ramifiés en C1-C4 ;
• un groupement hydroxyle,
• un radical alcoxy, linéaire ou ramifié, en C1-C4,
• un radical amino,
• un radical amino substitué par un ou plusieurs radicaux, identiques ou différents, choisis parmi les radicaux alkyle en C1-C6, linéaires ou ramifiés, éventuellement substitués par un ou plusieurs radicaux amino, hydroxyle, ammonium ou alkyl(C1-C4)ammonium, imidazolium, pyrazolium, ou pyridinium, amino substitué par un ou plusieurs radicaux, identiques ou différents, alkyle en C1-C4 linéaires ou ramifiés, alcoxy en C1-C4 linéaires ou ramifiés,
• un radical amino substitué dont les radicaux forment un hétérocycle à 5 ou 6 chaînons, saturé ou insaturé, comprenant éventuellement autre hétéroatome choisi parmi l'oxygène, l'azote ; ledit hétérocycle étant éventuellement substitué ;
• **R9, R10,** identiques ou non, ne pouvant représenter simultanément un atome de chlore ou de fluor ;
**X** représente un atome d'azote, -CR11 ; **R11** représentant :
• un atome d'hydrogène,
• un atome d'halogène, de préférence le chlore, le fluor,
• un groupement nitro, cyano,
• un radical alkyle, linéaire ou ramifié, en C1-C4, éventuellement porteur d'au moins un groupement hydroxyle, au moins un groupement alcoxy linéaire ou ramifié en C1-C4, au moins un groupement amino et/ou au moins un groupement amino substitué par un ou deux radicaux alkyles, identiques ou non, linéaires ou ramifiés en C1-C4,
• un radical alcoxy, linéaire ou ramifié, en C1-C4,
• un radical amino,
• un radical amino substitué par un ou deux radicaux, identiques ou différents, choisis parmi les radicaux alkyle en C1-C6, linéaires ou ramifiés, éventuellement substitués par un ou plusieurs radicaux amino, hydroxyle, amino substitué par un ou deux radicaux, identiques ou différents, alkyle en C1-C4 linéaires ou ramifiés, hydroxyalkyle en C1-C4 linéaires ou ramifiés,
• un radical amino substitué dont les radicaux forment un hétérocycle à 5 ou 6 chaînons, saturé ou insaturé, comprenant éventuellement autre hétéroatome choisi parmi l'oxygène, l'azote ; ledit hétérocycle étant éventuellement substitué ;
**L1,** cationique ou non, représente une chaîne hydrocarbonée en C₂-C₂₀, linéaire ramifiée ou cyclique, saturée ou insaturée, éventuellement interrompue ou terminée par un groupement comprenant un ou plusieurs hétéroatomes, éventuellement interrompue par un ou plusieurs (hétéro)cycles, aromatiques ou non, substitués ou non ;
**L2,** cationique ou non, représente :
• une chaîne hydrocarbonée en C₁-C₂₀, linéaire ramifiée ou cyclique, saturée ou insaturée, éventuellement interrompue ou terminée par un groupement comprenant un ou plusieurs hétéroatomes, éventuellement interrompue ou terminée par un ou plusieurs (hétéro)cycles, aromatiques ou non éventuellement substitués ;
• un radical (hétéro)cyclique, aromatique ou non, éventuellement rattaché aux atomes d'azote au moyen d'un groupement comprenant au moins un hétéroatome, de préférence un groupement CO, SO₂;
**L3**, cationique ou non, représente une chaîne hydrocarbonée en C₂-C₂₀, linéaire ramifiée ou cyclique, saturée ou insaturée, éventuellement interrompue ou terminée par un groupement comprenant un ou plusieurs hétéroatomes, éventuellement interrompue par un ou plusieurs (hétéro)cycles, aromatiques ou non éventuellement substitués ;
le coefficient **n** étant inférieur ou égal à 2 ;
le coefficient **n'** étant inférieur ou égal à 3 ;
le coefficient **m** étant inférieur ou égal à 4 ;
le coefficient **r** étant égal à 0 ou 1 ;
le coefficient **s** étant égal à 0 ou 1 ;
**An** représente un ou plusieurs contre ions cosmétiquement acceptables permettant d'assurer l'électroneutralité des composés de formule (I) et (II) ou de leurs sels d'addition.

2. Composés selon l'une quelconque des revendications précédentes, **caractérisés en ce que** les radicaux **R1, R2, R3,** identiques ou différents, représentent un radical alkyle en C1-C4, linéaire ou ramifié, substitué ou non par un groupement hydroxyle ; un radical alcoxy, linéaire ou ramifié, en C1-C4 ; un radical amino ; un radical amino substitué par un ou deux radicaux, identiques ou différents, alkyle en C1-C4, linéaire ou ramifié, hydroxyalkyle en C1-C4, linéaire ou ramifié.

3. Composés selon la revendication précédente, **caractérisés en ce que** les radicaux **R1, R2, R3,** identiques ou différents, représentent un radical alkyle en C1-C4, linéaire substitué ou non par un groupement hydroxyle.

4. Composés selon l'une quelconque des revendications précédentes, **caractérisés en ce que** les radicaux **R4, R'4, R5**, des formules (I) et (II), ces derniers, identiques ou différents, représentent :
• un atome d'halogène, de préférence le chlore,
• un groupement hydroxyle,
• un groupement nitro,
• un radical alkyle, linéaire ou ramifié, en C1-C4, éventuellement substitué par un ou plusieurs groupements hydroxyle, alcoxy en C1-C4 linéaires ou ramifiés,
• un radical alcoxy, linéaire ou ramifié, en C1-C4,
• un radical amino,
• un radical amino substitué par un ou deux radicaux, identiques ou différents, choisis parmi les radicaux alkyle en C1-C4, linéaires ou ramifiés, éventuellement substitués par un ou plusieurs groupements hydroxyle, radicaux amino, radicaux amino substitués par un ou deux radicaux, identiques ou différents, linéaires ou ramifiés, alkyle en C1-C4, hydroxyalkyle en C1-C4 ; par un groupement ammonium ou alkyl(C1-C4)ammonium ; par un groupement -SO₂NHR dans lequel R représente un atome d'hydrogène, un radical alkyle en C1-C4,
• un groupement -SO₂NHR dans lequel R représente un atome d'hydrogène, un radical alkyle en C1-C4.

5. Composés selon la revendication précédente, **caractérisés en ce que** les radicaux **R4, R'4, R5,** représentent :
• un atome d'halogène, de préférence le chlore,
• un groupement hydroxyle,
• un groupement nitro,
• un radical alkyle, linéaire en C1-C2, éventuellement substitué par un ou plusieurs groupements hydroxyle,
• un radical alcoxy, linéaire en C1-C2.

6. Composés selon l'une quelconque des revendications précédentes, **caractérisés en ce que** les radicaux **R6, R'6, R'7,** identiques ou différents, représentent un atome d'hydrogène ; un radical alkyle en C1-C4, linéaire ou ramifié, substitué ou non par un groupement hydroxyle, par un groupement alcoxy en C1-C4.

7. Composés selon la revendication précédente, **caractérisés en ce que** les radicaux **R6, R'6, R'7,** identiques ou différents, représentent un atome d'hydrogène, un radical alkyle en C1-C4, linéaire.

8. Composés selon l'une quelconque des revendications précédentes, **caractérisés en ce que** les radicaux **R9, R10,** identiques ou non, ceux-ci représentent :
• un atome d'hydrogène,
• un atome d'halogène, de préférence le chlore, le fluor;
• un groupement nitro, cyano ;
• un groupement hydroxyle
• un radical alcoxy, linéaire ou ramifié, en C1-C4,
• un radical amino ;
• un radical amino substitué par un ou deux radicaux, identiques ou différents, choisis parmi les radicaux alkyle en C1-C6, linéaires ou ramifiés, éventuellement substitués par un ou plusieurs groupements hydroxyle, radicaux amino, radicaux amino substitués par un ou deux radicaux, identiques ou différents, linéaires ou ramifiés, alkyle en C1-C4, hydroxyalkyle en C1-C4 ; un groupement ammonium ou alkyl(C1-C4)ammonium, imidazolium, pyrazolium ou pyridinium ;
• un radical amino substitué dont les radicaux forment un hétérocycle à 5 ou 6 chaînons, insaturé, comprenant éventuellement autre hétéroatome choisi parmi l'oxygène, l'azote ; ledit hétérocycle étant éventuellement substitué.

9. Composés selon la revendication précédente, **caractérisés en ce que** les radicaux **R9, R10,** identiques ou non, représentent :
• un atome d'hydrogène,
• un atome d'halogène, de préférence le chlore, le fluor,
• un groupement hydroxyle,
• un radical amino,
• un radical amino substitué par un ou deux radicaux, identiques choisis parmi les radicaux alkyle en C1-C4, linéaires, éventuellement substitués par un hydroxyle, amino substitué par un ou deux radicaux, identiques, alkyle en C1-C4 linéaires, un groupement ammonium ou alkyl(C1-C4)ammonium ou imidazolium ;
• un radical amino substitué dont les radicaux forment un hétérocycle à 5 ou 6 chaînons, insaturé, comprenant éventuellement un azote ; ledit hétérocycle étant éventuellement substitué.

10. Composés selon l'une quelconque des revendications précédentes, **caractérisés en ce que** le radical **R11** représente :
• un atome d'halogène, de préférence le chlore, le fluor;
• un groupement nitro, cyano,
• un groupement hydroxyle,
• un radical alcoxy, linéaire ou ramifié, en C1-C4,
• un radical amino,
• un radical amino substitué par un ou deux radicaux, identiques ou différents, choisis parmi les radicaux alkyle en C1-C6, linéaires ou ramifiés, éventuellement substitués par un ou plusieurs groupements hydroxyle, radicaux amino, radicaux amino substitués par un ou deux radicaux, identiques ou différents, linéaires ou ramifiés, alkyle en C1-C4, hydroxyalkyle en C1-C4,
• un radical amino substitué dont les radicaux forment un hétérocycle à 5 ou 6 chaînons, insaturé, comprenant éventuellement autre hétéroatome choisi parmi l'oxygène, l'azote ; ledit hétérocycle étant éventuellement substitué.

11. Composés selon la revendication précédente, **caractérisés en ce que** lorsque le radical **R11** représente un radical amino substitué dont les radicaux forment un hétérocycle à 5 ou 6 chaînons, l'hétérocycle est choisi parmi la pyrrolidine, pipéridine, homopipéridine, pipérazine, homopipérazine, diazépane. De plus l'hétérocycle peut éventuellement être substitué.

12. Composés selon la revendication précédente, **caractérisés en ce que** l'hétérocycle est choisi parmi la pyrrolidine, la 3-hydroxypyrrolidine, la 3-aminopyrrolidine, la 3-N,N-diméthylaminopyrrolidine, la 3-triméthylammoniumpyrrolidine, la 3-acétamidopyrrolidine, la 3-(méthylsulfonylamino)-pyrrolidine, la proline, la 3-hydroxyproline, la pipéridine, l'hydroxypipéridine, l'homopipéridine, le diazépane, N-méthyl homopipérazine, la N β-hydroxyéthylhomopipérazine.

13. Composés selon l'une quelconque des revendications précédentes, **caractérisés en ce que** le coefficient r vaut 1 et le groupement **L1 ou L3** représente une chaîne alkylène en C1-C20, linéaire ou ramifiée, substituée ou non, éventuellement interrompue par au moins un atome d'oxygène, par au moins un groupement -NR-avec R représentant un atome d'hydrogène, un radical alkyle, linéaire ou ramifié en C1-C4, hydroxyalkyle, linéaire ou ramifié en C1-C4, par au moins un groupement - N⁺(R')(R")- avec R', R", identiques ou non, un radical alkyle, linéaire ou ramifié en C1-C4, hydroxyalkyle, linéaire ou ramifié en C1-C4, par au moins un groupement carbonyle, par au moins un (hétéro)cycle, aromatique ou non, cationique ou non, éventuellement substitué ; éventuellement terminée par un groupement carbonyle.

14. Composés selon la revendication précédente, **caractérisés en ce que**, le groupement **L1 ou L3** représente une chaîne alkylène en C1-C20, de préférence en C1-C8, interrompue par au moins un (hétéro)cycle, aromatique ou non, cationique ou non, éventuellement substitué choisi parmi les hétérocycles pipérazine, homopipérazine, diazépane, pyrrolidine, (homo)pipéridine, triazine.

15. Composés selon l'une quelconque des revendications13 ou 14, **caractérisés en ce que L1, L3,** représentent une chaîne alkylène en C1-C8, linéaire, substituée ou non ; éventuellement interrompue par au moins un atome d'oxygène, par au moins un groupement -NR- avec R représentant un atome d'hydrogène, un radical alkyle, linéaire ou ramifié en C1-C4, hydroxyalkyle, linéaire ou ramifié en C1-C4, par au moins un groupement -N⁺(R')(R")- avec R', R", identiques ou non, un radical alkyle, linéaire ou ramifié en C1-C4, hydroxyalkyle, linéaire ou ramifié en C1-C4, par au moins un hétérocycle éventuellement substitué choisi parmi la pipérazine, la pyrrolidine, la triazine ; imidazolium, éventuellement terminée par un groupement carbonyle.

16. Composés selon l'une quelconque des revendications13 à 15, **caractérisés en ce que L1, L3,** représentent une chaîne alkylène en C1-C8, linéaire, éventuellement interrompue par au moins un atome d'oxygène, au moins un groupement -NR- avec R représentant un atome d'hydrogène, un radical alkyle, linéaire en C1-C4, au moins un groupement -N(R')(R")⁺- avec R', R", identiques, un radical alkyle, linéaire en C1-C4.

17. Composés selon l'une quelconque des revendications précédentes, **caractérisés en ce que** le coefficient r est égal à 0.

18. Composés selon l'une quelconque des revendications précédentes, **caractérisés en ce que** le coefficient s est égal à 1 et **L2** représente :
• une chaîne alkylène en C1-C20, linéaire ou ramifiée, substituée ou non, éventuellement interrompue par au moins un atome d'oxygène, au moins un groupement -NR- avec R représentant un atome d'hydrogène, un radical alkyle, linéaire ou ramifié en C1-C4, hydroxyalkyle, linéaire ou ramifié en C1-C4, au moins un groupement -N(R')(R")⁺- avec R', R", identiques ou non, un radical alkyle, linéaire ou ramifié en C1-C4, hydroxyalkyle, linéaire ou ramifié en C1-C4, éventuellement interrompue ou terminée par au moins un groupement carbonyle, au moins un (hétéro)cycle, aromatique ou non, cationique ou non, éventuellement substitué ;
• un phénylène ;
• un hétérocycle à 6 ou 7 chaînons, saturé ou non, comprenant au moins deux hétéroatomes, le radical R6 et l'atome d'azote qui le porte étant engagés dans ledit hétérocycle.

19. Composés selon la revendication précédente, **caractérisés en ce que L2** représente une chaîne alkylène en C1-C20, de préférence en C1-C8, interrompue ou terminée par au moins un (hétéro)cycle, aromatique ou non, substitué ou non, choisi parmi les hétérocycles pipérazine, homopipérazine, diazépane, triazine

20. Composés selon l'une des revendications 18 ou 19, **caractérisés en ce que L2** représente :
• une chaîne alkylène en C1-C8, linéaire, substituée ou non, éventuellement interrompue par au moins un atome d'oxygène, au moins un groupement -NR-avec R représentant un atome d'hydrogène, un radical alkyle, linéaire ou ramifié en C1-C4, hydroxyalkyle, linéaire ou ramifié en C1-C4, au moins un groupement - N(R')(R")⁺- avec R', R", identiques ou non, un radical alkyle, linéaire ou ramifié en C1-C4, hydroxyalkyle, linéaire ou ramifié en C1-C4, pipérazinium, homopipérazinium, imidazolium, éventuellement terminée par au moins un groupement carbonyle, au moins un hétérocycle, aromatique ou non, éventuellement substitué;
• un phénylène ;
• avec le radical R6 et l'atome d'azote qui le porte, un hétérocycle pipérazine ou homopipérazine non substitué.

21. Composés selon l'une des revendications 18 à 20 **caractérisés en ce que L2** représente :
• une chaîne alkylène en C1-C8, linéaire, éventuellement interrompue par au moins un atome d'oxygène, au moins un groupement -NR- avec R représentant un atome d'hydrogène, un radical alkyle, linéaire en C1-C4, au moins un groupement -N⁺(R')(R")- avec R', R", identiques, un radical alkyle, linéaire en C1-C4, imidazolium, éventuellement terminée par au moins un hétérocycle, aromatique ou non, éventuellement substitué, de préférence triazine éventuellement substitué ;
• un phénylène ;
• avec le radical R6 et l'atome d'azote qui le porte, un hétérocycle pipérazine ou homopipérazine non substitué.

22. Composés selon l'une quelconque des revendications précédentes, **caractérisés en ce que L2** représente un groupement de formule (d) suivante : 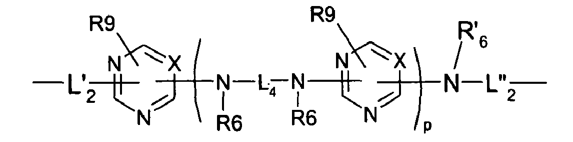 formule dans laquelle :
**R1, R2, R3, R4, R5, R6, R'6, R9, X,** ayant la même signification que précédemment ;
**L'2, L"2**, identiques ou non, représentent une liaison simple, une chaîne alkylène en C1-C6; L'2 étant rattaché au groupement NR6,
**L4** représente une chaîne alkylène en C1-C20, linéaire ou ramifiée, substituée ou non, éventuellement interrompue par au moins un atome d'oxygène, par au moins un groupement -NR- avec R représentant un atome d'hydrogène, un radical alkyle, linéaire ou ramifié en C1-C4, hydroxyalkyle, linéaire ou ramifié en C1-C4, par au moins un groupement -N⁺(R')(R")- avec R', R", identiques ou non, représentant un radical alkyle, linéaire ou ramifié en C1-C4, hydroxyalkyle, linéaire ou ramifié en C1-C4,
le coefficient **p** étant égal à 0 ou 1.

23. Composés selon l'une quelconque des revendications précédentes, **caractérisés en ce que** le contre ion cosmétiquement acceptable est choisi parmi les sels d'acides minéraux comme par exemple les chlorures, les bromures, les iodures, les sulfates, hydrogénosulfates, les phosphates ; parmi les sels d'acides organiques comme les formiates, les acétates, les citrates, les succinates, les tartrates, les lactates, les tosylates, les mésylates, les benzènesulfonates, les alkylsulfates et, ou leurs mélanges.

24. Composés selon l'une quelconque des revendications précédentes, de formule suivante (III) ou ses sels : 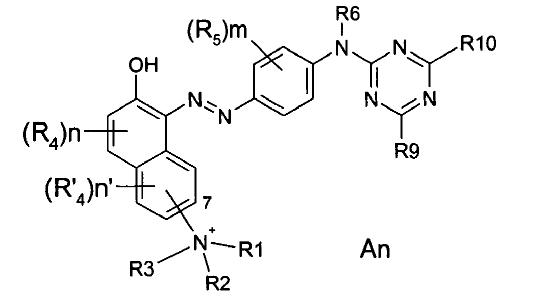 Formule dans laquelle les radicaux **R1, R2, R3, R4, R'4, R5, R6, R9, R10,** et les coefficients n et n' ont les mêmes significations générales et plus particulières indiquées auparavant.

25. Composés selon la revendication précédente, **caractérisés en ce que** :
**R1, R2, R3,** identiques ou différents, représente un radical alkyle linéaire ou ramifié en C1-C4,
**R4, R'4,** représentent un tome d'hydrogène,
**R5** représente un atome d'hydrogène, un groupement nitro,
**R6,** représente un atome d'hydrogène, un radical alkyle linéaire ou ramifié en C1-C4, de préférence méthyle,
**R9, R10,** identiques ou non, représentent un atome d'hydrogène, un atome de chlore, un atome de fluor, un radical hydroxyle, un radical amino, un radical amino substitué par un ou deux radicaux, identiques ou différents, linéaires ou ramifiés, alkyle en C1-C4, hydroxyalyle en C1-C4, un cycle pyrrolidine éventuellement substitué par un radical trialkyl(C1-C4)ammonium avec des radicaux alkyle, identiques ou non, linéaires ou ramifiés, en C1-C4, de préférence méthyle,
**R9 et R10,** identiques ou non, ne représentant pas simultanément un atome de chlore ou un atome de fluor,
l'électroneutralité des composés de formule (III) ou leurs sels étant assurée par un ou plusieurs contre ions **An,** identiques ou différents, cosmétiquement acceptables,
le groupement ammonium se trouvant en position 7 sur le noyau naphtalène.

26. Composés selon l'une quelconque des revendications précédentes, de formules suivantes, ou leurs sels correspondants : 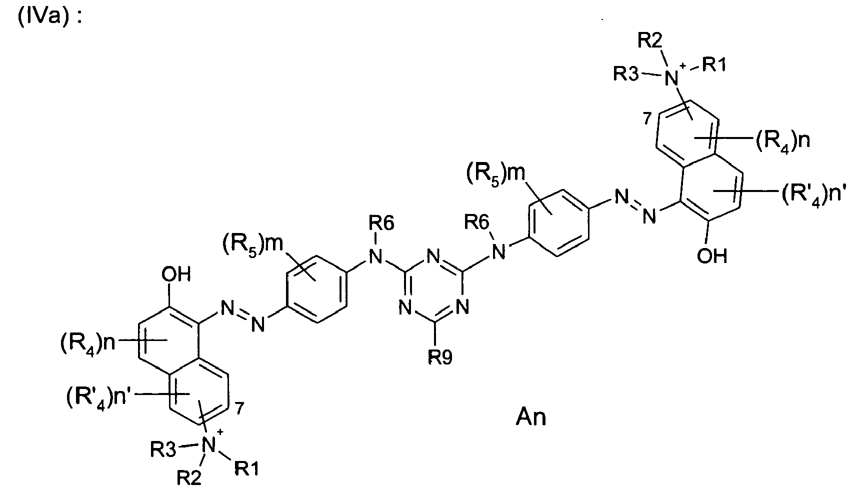 Formule dans laquelle les radicaux **R1, R2, R3, R4, R'4, R5, R6, R9,** et les coefficients **n** et **n'** ont les mêmes significations que précédemment ; 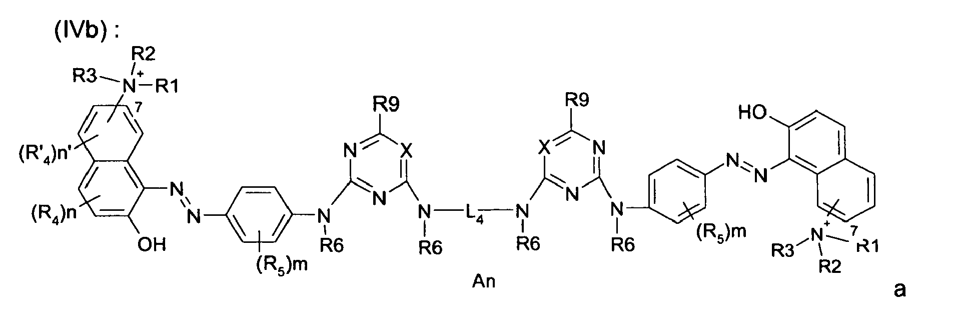 Formule dans laquelle les radicaux **R1, R2, R3, R4, R'4, R5, R6, R9,** et les coefficients n et n' ont les mêmes significations que précédemment.

27. Composés selon la revendication précédente, **caractérisé en ce que** les radicaux :
**R1, R2, R3,** identiques ou différents, représente un radical alkyle linéaire ou ramifié en C1-C4,
**R4, R'4,** représentent un tome d'hydrogène,
**R5** représente un atome d'hydrogène, un groupement nitro ;
**R6**, représente un atome d'hydrogène, un radical alkyle linéaire ou ramifié en C1-C4, de préférence méthyle,
**R9** représente un atome d'hydrogène, un atome de chlore, un atome de fluor, un radical hydroxyle, un radical amino, un radical amino substitué par un ou deux radicaux, identiques ou différents, linéaires ou ramifiés, alkyle en C1-C4, hydroxyalkyle en C1-C4, un cycle pyrrolidine éventuellement substitué par un radical trialkyl(C1-C4)ammonium avec des radicaux alkyle, identiques ou non, linéaires ou ramifiés, en C1-C4, de préférence méthyle ;
l'électroneutralité des composés de formule (IV) ou leurs sels étant assurée par un ou plusieurs contre ions **An**, identiques ou non, cosmétiquement acceptables, choisis notamment parmi ceux listés auparavant.
le groupement ammonium des formules (IVa) et (IVb) se trouvant en position 7 sur le noyau diaromatique.

28. Composés selon l'une quelconque des revendications précédentes, de formule suivante (V) : 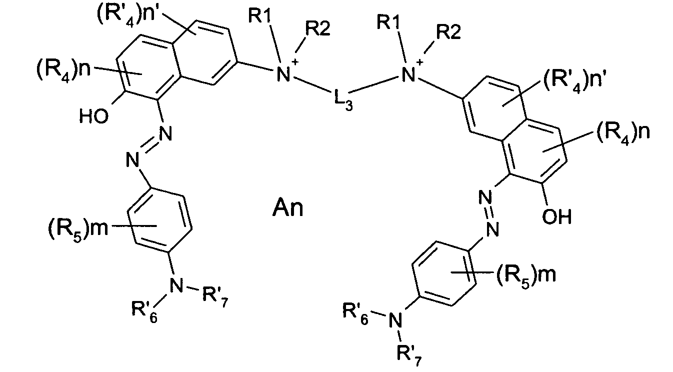 Formule dans laquelle les radicaux **R4, R'4, R5, R'6, R'7, R1, R2** et les coefficients **n** et **n'** ont les mêmes significations que précédemment.

29. Composés selon la revendication précédente, **caractérisé en ce que** les radicaux :
**R4, R'4,** représentent un atome d'hydrogène,
**R5** représente un atome d'hydrogène, un groupement nitro ;
**R'6, R'7,** représentent un atome d'hydrogène ;
**R12, R13,** identiques ou différents, représentent un atome d'hydrogène, un radical alkyle, linéaire ou ramifié, en C1-C4, un radical hydroxyalkyle linéaire ou ramifié, en C1-C4 ;
**L3** représente un radical alkylène, linéaire ou ramifié, comprenant de 1 à 10 atomes de carbone, et de préférence de 2 à 8 atomes de carbone ;
l'électroneutralité des composés de formule (V) ou leurs sels étant assurée par un ou plusieurs contre ions **An,** identiques ou différents, cosmétiquement acceptables.

30. Procédé de préparation de composés selon l'une quelconque des revendications précédentes, dans lequel on met en contact un composé de formule (VI) 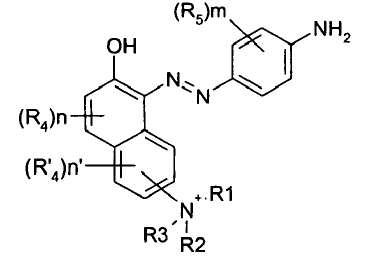 avec un halogénure cyanurique, en présence d'un solvant ou d'un mélange de solvant pour obtenir un composé de formule (I) dans lequel R7 représente un groupe de formule (a).

31. Procédé de préparation de composés selon l'une quelconque des revendications précédentes, dans lequel on met en contact un composé de formule (VI) 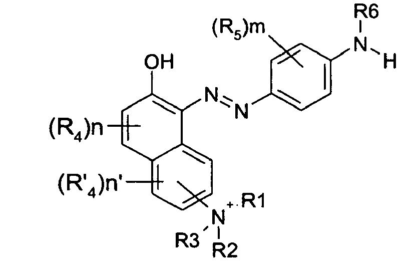 avec un composé précurseur de L2 ou avec un composé comprenant L2 et deux fonctions susceptibles de réagir avec les fonctions amine du composé (VI), en présence d'un solvant ou d'un mélange de solvant, pour obtenir un composé de formule (I) dans lequel R7 représente un groupe de formule (b).

32. Procédé selon la revendication précédente, **caractérisé en ce que** l'on met en contact les quantités appropriées en composé de formule (VI) et d'halogénure cyanurique.

33. Procédé selon la revendication 31, **caractérisé en ce que** l'on met en oeuvre els étapes suivantes :
(a) on met contact un composé de formule (VII) suivante : 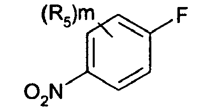 avec une diamine de formule R₆NH-L₂-NHR₆ pour obtenir un composé de formule (VIII) suivante : 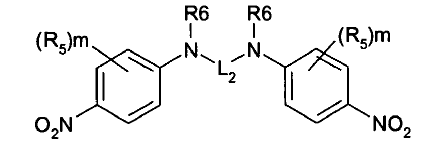
(b) on effectue une étape de réduction des groupements des groupements nitro du composé ainsi obtenu, en présence d'hydrogène et d'un catalyseur,
(c) on effectue une diazotation en mettant en contact le produit résultant pour obtenir le composé de formule (VIII) suivante : 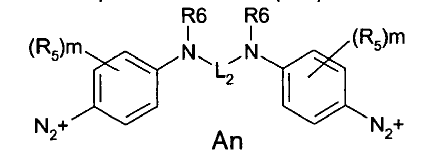
(d) on met en contact le produit (VIII) avec un composé de formules (IXa) ou (IXb) suivantes : 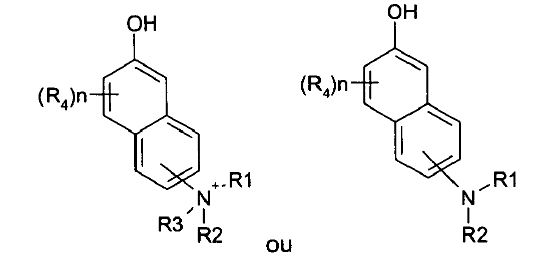
Au cas où l'on utilise le composé comprenant la fonction amine (IXb), on effectue une étape supplémentaire, de quaternisation du produit résultant ;

34. Procédé de préparation de composés selon l'une quelconque des revendications précédentes, dans lequel on met en oeuvre els étapes suivantes :
(a) on met en contact un composé de formule (X) suivante : 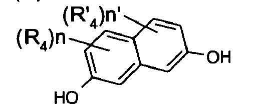 avec une diamine de formule H₂N-L₃-NH₂ et l'on obtient un composé de formule suivante (XI) : 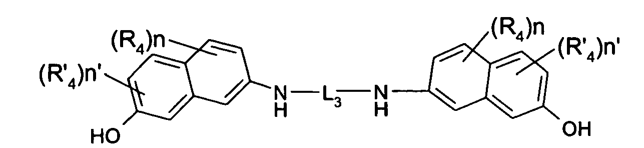
(b) on effectue une N-alkylation pui une quaternisation du produit obtenu,
(c) on fait réagir le produit résultant avec un composé de formule (XII) suivante : 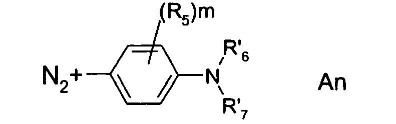
et l'on obtient un composé de formule (II).

35. Composition tinctoriale comprenant, dans un milieu approprié pour la teinture des fibres kératiniques, au moins un colorant direct choisi parmi les composés selon l'une quelconque des revendications 1 à 29.

36. Composition selon la revendication précédente, **caractérisée en ce que** la teneur en chacun des composés de formules (I) et (II) ou leurs sels, varie de 0,001 à 20 % en poids par rapport au poids de la composition tinctoriale.

37. Procédé de coloration de fibres kératiniques dans lequel on applique sur des fibres sèches ou humides, une composition tinctoriale selon l'une quelconque des revendications 35 ou 36, sans rinçage final.

38. Procédé de coloration de fibres kératiniques dans lequel on applique sur des fibres sèches ou humides, une composition tinctoriale selon l'une quelconque des revendications 35 ou 36, pendant une durée suffisante au développement de la coloration, après quoi on rince et/ou on lave au shampooing les fibres.
